(19) Europäisches Patentamt
European Patent Office
Office européen des brevets

(11) **EP 2 519 243 B1**

(12) **EUROPEAN PATENT SPECIFICATION**

(45) Date of publication and mention of the grant of the patent:
**22.04.2015 Bulletin 2015/17**

(21) Application number: **10841736.1**

(22) Date of filing: **30.12.2010**

(51) Int Cl.:
***A61K 31/7068*** *(2006.01)* ***A61K 31/506*** *(2006.01)*
***A61K 31/675*** *(2006.01)* ***A61P 31/20*** *(2006.01)*
***A61K 31/513*** *(2006.01)*

(86) International application number:
**PCT/US2010/062547**

(87) International publication number:
**WO 2011/082331 (07.07.2011 Gazette 2011/27)**

(54) **COMPOSITIONS AND METHODS FOR TREATING HEPATITIS B VIRUS INFECTION**

ZUSAMMENSETZUNGEN UND VERFAHREN ZUR BEHANDLUNG EINER HEPATITIS-B-VIRUSINFEKTION

COMPOSITIONS ET MÉTHODES POUR TRAITER UNE INFECTION PROVOQUÉE PAR LE VIRUS DE L'HÉPATITE B

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.12.2009 US 291728 P**

(43) Date of publication of application:
**07.11.2012 Bulletin 2012/45**

(73) Proprietor: **Zhou, Xiao-Jian**
**Arlington, MA 02474 (US)**

(72) Inventor: **Zhou, Xiao-Jian**
**Arlington, MA 02474 (US)**

(74) Representative: **Duffy, Assumpta Dympna**
**FRKelly**
**27 Clyde Road**
**Ballsbridge**
**Dublin 4 (IE)**

(56) References cited:
**WO-A2-00/16755**
**CN-A- 101 229 178**
**JP-A- 2009 159 845**
**US-A1- 2008 161 256**
**US-A1- 2008 226 596**

- **CAREY IVANA ET AL: "EFFICACY AND SAFETY OF LAMIVUDINE PLUS ADEFOVIR DE NOVO COMBINATION THERAPY COMPARED TO ENTECAVIR MONOTHERAPY IN CHRONIC HEPATITIS B: A SINGLE CENTRE CLINICAL PRACTICE EXPERIENCE", HEPATOLOGY, vol. 50, no. 4, Suppl. S, October 2009 (2009-10), page 502A, XP002694881, & 60TH ANNUAL MEETING OF THE AMERICAN-ASSOCIATION-FOR-THE-STUDY-OF-LIVE R-DISEASES; BOSTON, MA, USA; OCTOBER 30 -NOVEMBER 03, 2009 ISSN: 0270-9139**
- **TORRE FRANCESCO ET AL: "INITIAL HIGH DOSE OF LAMIVUDINE DELAYS THE APPEARANCE OF VIRAL RESISTANCE IN CHRONIC HEPATITIS B PATIENTS", HEPATOLOGY; 60TH ANNUAL MEETING OF THE AMERICAN-ASSOCIATION-FOR-THE-STUDY-OF-LIVE R-DISEASES, WILEY, USA; BOSTON, MA, USA , vol. 50, no. 4, Suppl 3 October 2009 (2009-10-03), pages 524A-525A, XP008161172, ISSN: 0270-9139, DOI: 10.1002/HEP.23302 Retrieved from the Internet: URL:http: //www3.interscience.wiley.com/cgi -bin/issn? DESCRIPTOR=PRINTISSN&VALUE=0270- 9139 [retrieved on 2009-09-23]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- C. C. WANG ET AL: "Kinetics of hepatitis B viral load during 48 weeks of treatment with 600 mg vs 100 mg of lamivudine daily*", JOURNAL OF VIRAL HEPATITIS, vol. 11, no. 5, 1 September 2004 (2004-09-01), pages 443-447, XP055058395, ISSN: 1352-0504, DOI: 10.1111/j.1365-2893.2004.00523.x
- BENHAMOU Y ET AL: "Effects of lamivudine on replication of hepatitis B virus in HIV-infected men.", ANNALS OF INTERNAL MEDICINE 1 NOV 1996, vol. 125, no. 9, 1 November 1996 (1996-11-01), pages 705-712, XP008161190, ISSN: 0003-4819
- LAI CHING-LUNG ET AL: "Lamivudine is effective in suppressing hepatitis B virus DNA in Chinese hepatitis B surface antigen carriers: A placebo-controlled trial", HEPATOLOGY, vol. 25, no. 1, 1997, pages 241-244, ISSN: 0270-9139
- NEVENS F ET AL: "LAMIVUDINE THERAPY FOR CHONIC HEPATITIS B: A SIX-MONTH RANDOMIZED DOSE-RANGING STUDY", GASTROENTEROLOGY, ELSEVIER, PHILADELPHIA, PA, vol. 113, no. 4, 1 January 1997 (1997-01-01), pages 1258-1263, XP001066327, ISSN: 0016-5085, DOI: 10.1053/GAST.1997.V113.PM9322520
- THIBAULT VINCENT ET AL: "Hepatitis B virus (HBV) mutations associated with resistance to lamivudine in patients coinfected with HBV and human immunodeficiency virus", JOURNAL OF CLINICAL MICROBIOLOGY, vol. 37, no. 9, September 1999 (1999-09), pages 3013-3016, ISSN: 0095-1137
- LOK ANNA S F ET AL: "AASLD guidelines on chronic hepatitis B (vol 45, pg 507, 2007)", HEPATOLOGY, vol. 45, no. 6, June 2007 (2007-06), page 1347, ISSN: 0270-9139
- HEZODE C ET AL: "Efficacy and safety of adefovir dipivoxil 20 mg daily in HBeAg-positive patients with lamivudine-resistant hepatitis B virus and a suboptimal virological response to adefovir dipivoxil 10 mg daily", JOURNAL OF HEPATOLOGY 200705 NL, vol. 46, no. 5, May 2007 (2007-05), pages 791-796, ISSN: 0168-8278
- QAQISH R B ET AL: "Adefovir Dipivoxil: A New Antiviral Agent for the Treatment of Hepatitis B Virus Infection", CLINICAL THERAPEUTICS 200312 US, vol. 25, no. 12, December 2003 (2003-12), pages 3084-3099, ISSN: 0149-2918
- TERRAULT N A: "Benefits and risks of combination therapy for hepatitis B", HEPATOLOGY 2009 JOHN WILEY AND SONS LTD USA, vol. 49, no. SUPPL. 5, May 2009 (2009-05), pages S122-S128, ISSN: 0270-9139
- JOHNSON M A ET AL: "Clinical pharmacokinetics of lamivudine", CLINICAL PHARMACOKINETICS 1999 NZ, vol. 36, no. 1, 1999, pages 41-66, ISSN: 0312-5963
- LOK A S F ET AL: "Chronic hepatitis B: Update 2009", HEPATOLOGY 2009 JOHN WILEY AND SONS LTD USA, vol. 50, no. 3, 2009, pages 661-662, ISSN: 0270-9139
- SUNG J J Y ET AL: "Lamivudine compared with lamivudine and adefovir dipivoxil for the treatment of HBeAg-positive chronic hepatitis B", JOURNAL OF HEPATOLOGY 200805 NL, vol. 48, no. 5, May 2008 (2008-05), pages 728-735, ISSN: 0168-8278

## Description

### Technical Field of the Invention

The invention generally relates to compositions and their use in treating hepatitis B virus infection. More particularly, the invention relates to treating hepatitis B virus infection and related conditions in humans using unique and synergistic combinations of lamivudine and adefovir that are designed to maximize favorable therapeutic outcomes while minimizing or preventing viral resistance, which commonly occurs from using lamivudine or adefovir alone, and reducing the side effects of adefovir.

### Background of the Invention

Hepatitis B is a disease caused by the hepatitis B virus (HBV). HBV infection of the liver of hominoidae, including humans, causes hepatitis, which is an inflammation in the liver. Hepatitis B is a major healthcare issue worldwide and is very common (pandemic) in parts of Asia and Africa in particular. In China, for example, about 7% of the overall population are chronically infected with HBV, and are persistently seropositive for HBV surface antigen (HBsAg); such persons are termed "HBsAg carriers" (Data on 2006 seroepidemiological survey on HBV infection in China). According to the World Health Organization (WHO), there are an estimated 350 million people with chronic HBV infection worldwide. Chronic hepatitis B (CHB) is a medical term that refers to a chronic, potentially progressive inflammatory liver disease associated with chronic HBV infection. Chronic hepatitis B may eventually cause liver cirrhosis, which can result in premature mortality from liver failure and liver cancer, a potentially fatal disease with a very poor response to current therapy. (Chang, M. "Hepatitis B virus infection" Seminars in fetal & neonatal medicine, 2007, 12 (3): 160-167.) According to the WHO, Hepatitis B causes about 600,000 deaths of HBV related liver failure, cirrhosis, and hepatocellular carcinoma annually.

HBV is a member of the Hepadnavirus family. The virus particle (called a "Dane particle") consists of an outer lipid envelope and an icosahedral nucleocapsid core that is composed of protein. The nucleocapsid encloses the viral DNA and a DNA polymerase that has reverse transcriptase activity. The outer envelope contains embedded proteins, which are involved in viral binding of and entry into susceptible cells.

The double-stranded DNA genome (chromosome) of the HBV virus replicates inside cells through an RNA intermediate by reverse transcription. HBV replication takes place primarily in liver cells. In HBV-infected persons, virus-specific proteins and their corresponding antibodies are found in the blood, allowing blood tests for these proteins and antibodies to be used for the diagnosis of HBV infection.

Several classes of medications are available to treat HBV infection. These include synthetic antiviral nucleosides and nucleotides such as lamivudine (Epivir-HBV/ Zeffix), adefovir dipivoxil (Hepsera), entecavir (Baraclude), telbivudine (Tyzeka/Sebivo) and tenofovir disoproxil fumarate (Viread), as well as immune system modulators such as interferon alpha-2b (Intron-A), interferon alpha-2a and pegylated interferon alpha-2a (Pegasys).

Lamivudine, a nucleoside analog, was approved by the U.S. FDA in 1998 as the first oral antiviral for the treatment of CHB. The chemical name of lamivudine is (2R,cis)-4- amino-1-(2-hydroxymethyl-1,3-oxathiolan-5-yl)-(1H)-pyrimidin-2-one. For treating HBV infection, lamivudine is most commonly taken orally once daily as a 100-mg tablet, the regulatory-approved dosage regimen for CHB patients.

Adefovir dipivoxil, an acyclic nucleotide monophosphate analog, was approved by the U.S. FDA for the treatment of HBV in 2002. The chemical name of adefovir dipivoxil is 9-[2-[[bis[(pivaloyloxy)methoxy]-phosphinyl]-methoxy]ethyl]adenine. It contains two pivaloyloxymethyl units, making it a pro-drug form of adefovir. Adefovir dipivoxil is most commonly taken orally once daily as a 10-mg tablet, the regulatory-approved dosage regimen.

Both lamivudine and adefovir require intracellular phosphorylation to their respective active tri- and di- phosphates, which competitively inhibit the HBV DNA polymerase and result in chain termination of nascent viral DNA.

Drug resistance can emerge after treatment of CHB patients with antiviral nucleosides and nucleotides, particularly during prolonged monotherapy. Although short-term therapy is feasible in certain subgroups of CHB patients, prolonged therapy of two years or longer is typically required for lasting viral suppression in most CHB patients. Drug-resistant variants of HBV emerge more frequently in patients with suboptimal suppression of HBV replication in the early months of treatment, due to continual amplification, under the selective pressure of drug treatment, of spontaneously-occurring viral populations that are resistant to the antiviral agent.

Development of drug resistance to lamivudine has been a major clinical obstacle to the long-term effectiveness of lamivudine in many patients. Viral species with mutations conferring resistance to lamivudine are detectable in some lamivudine-treated CHB patients within the first 3-6 months of treatment, and become increasingly common after that. The incidence of lamivudine resistance increases with duration of therapy up to 70% of all treated patients after five years of lamivudine therapy. (Liaw, J. Clinical Virology, vol. 34, Supp. 1, pp. S143-S146 (2005).) Drug resistance to lamivudine is primarily associated with mutations in HBV DNA sequences encoding a four amino-acid sequence (the

YMDD motif) located within the conserved catalytic domain of the viral polymerase/reverse transcriptase.

While viral resistance occurs at a much slower rate in the first year with adefovir dipivoxil, the cumulative rate of resistance to adefovir in 5 years is substantial, about 20% in HBeAg positive patients and 29% in HBeAg negative patients. (Marcellin et al. "Long-term efficacy and safety of adefovir dipivoxil for the treatment of hepatitis B e antigen-positive chronic hepatitis B" Hepatology 2008, 48(3):750-758; Hadziyannis et al. "Adefovir Dipivoxil 438 Study Group. Long-term therapy with adefovir dipivoxil for HBeAg-negative chronic hepatitis B for up to 5 years" Gastroenterology 2006, 131(6):1743-1751.)

Emergence of resistance to anti-HBV drugs can be associated with substantial increases in HBV replication, which is detected as rises in previously-suppressed serum HBV DNA levels with return of HBV DNA levels to easily-detectable levels, a phenomenon called viral breakthrough. Such recrudescence of HBV replication can be associated with worsening of the clinical severity of hepatitis, due to increased liver inflammation; in some patients the worsened liver inflammation can result in hepatic decompensation (liver failure). Also, the emergence of drug-resistant HBV strains is associated with reduced treatment benefit through reduced rates of HBeAg seroconversion (reduced clearance of HBV infection to clinically-insignificant levels). With the advent of newer agents with lower resistance rates, prolonged use of lamivudine monotherapy has been avoided. This has made lamivudine a suboptimal drug against HBV when used alone. Of note, despite the relatively high rate of resistance with prolonged lamivudine therapy, lamivudine use continues in some locales because it is an often-effective, and yet a low cost drug that is readily available to most HBV patients worldwide.

Due to the 20-30% 5-year resistance rate to adefovir, in recent years some clinicians have also reduced their use of adefovir monotherapy and have turned to newer agents with even lower rates of viral resistance, i.e. entecavir (Baraclude) and tenofovir dipivoxil (Viread).

It has been shown that mutants resistant to lamivudine retain susceptibility to adefovir, and vice versa. This has led to the approach of treating patients with lamivudine resistance by adding adefovir dipivoxil onto the lamivudine treatment regimen. Similarly, add-on lamivudine therapy has been used in patients who developed adefovir resistance. Published date indicated that, in anti-HBV treatment naive patients, however, *de novo* combination of lamivudine with adefovir dipivoxil has not to date resulted in additive or synergistic antiviral activity, for the comparison of results with lamivudine plus adefovir to lamivudine alone, and has failed to adequately prevent the emergence of resistance to lamivudine. (Lok et al. "AASLD practice guidelines. Chronic hepatitis B: update 2009" Hepatology 2009, 50(3): 1-35.)

With years of clinical experience in thousands of patients, both lamivudine and adefovir dipivoxil are regarded as generally safe and well tolerated. However, prolonged use or adefovir dipivoxil at the regulatory-approved dose of 10 mg per day is associated with a cumulative risk for nephrotoxicity that occurs at a rate of 3% in 4-5 years in patients with compensated liver disease. In patients with predisposing medical conditions such as decompensated liver disease and liver transplantation, adefovir-associated nephrotoxicity has been reported in up to 47% of patients, with treatment periods of 39-99 weeks. (Lok et al. "AASLD practice guidelines. Chronic hepatitis B: update 2009" Hepatology 2009, 50(3): 1 -35.)

WO 00/16755 discloses the combined use of lamivudine in a dosage of 25 to 150 mg and adefovir dipivoxil in a dosage of 5 to 60 mg in the treatment of an HBV infection.

Thus, pharmaceutical treatments that effectively treat chronic HBV infection with negligible viral resistance and minimal side effects with prolonged therapy are highly desirable. Additionally, it is much preferred that such pharmaceutical treatments are also readily available at affordable prices.

## Summary of the Invention

The invention is based, in part, on the unexpected discovery of unique and synergistic combinations of lamivudine and adefovir at doses that maximize therapeutic effects for the treatment of hepatitis B virus infection and related conditions in humans, while minimizing or preventing the onset of viral resistance to lamivudine or adefovir. Pharmaceutical compositions of the invention and related methods of treatment provide much-increased anti-viral activity while blocking the emergence of viral resistance.

In one aspect, the invention generally relates to a pharmaceutical composition for treating hepatitis B virus infection in a human. The pharmaceutical composition includes: a first active pharmaceutical ingredient consisting of more than 400 mg to about 1500 mg of lamivudine, (2R, cis)-4-amino-1-(2-hydroxymethyl-1,3-oxathiolan-5-yl)-pyrimidin-2-one, or a therapeutically equivalent amount of a pharmaceutically acceptable salt or ester thereof; and a second active pharmaceutical ingredient consisting of about 1 mg to about 10 mg of adefovir dipivoxil, bis(pivaloyloxymethyl)(9-[(R)-2(phosphonomethoxy)ethyl]adenine, or a therapeutically equivalent amount of a pharmaceutically acceptable salt or ester thereof.

In some embodiments of the invention, the first active ingredient consists of more than 400 mg to about 900 mg of lamivudine, or a therapeutically equivalent amount of a pharmaceutically acceptable salt or ester thereof; and the second active ingredient consists of about 2 mg to about 8 mg of adefovir dipivoxil, or a therapeutically equivalent amount

of a pharmaceutically acceptable salt or ester thereof.

In some preferred embodiments of the invention, the first active ingredient consists of about 600 mg to about 900 mg of lamivudine, or a therapeutically equivalent amount of a pharmaceutically acceptable salt or ester thereof; and the second active ingredient consists of about 2 mg to about 5 mg of adefovir dipivoxil, or a therapeutically equivalent amount of a pharmaceutically acceptable salt or ester thereof.

In some preferred embodiments of the invention, the first active ingredient consists of more than 400 mg to about 600 mg of lamivudine, or a therapeutically equivalent amount of a pharmaceutically acceptable salt or ester thereof; and the second active ingredient consists of about 5 mg to about 8 mg of adefovir dipivoxil, or a therapeutically equivalent amount of a pharmaceutically acceptable salt or ester thereof.

In some preferred embodiments of the invention, the first active ingredient consists of about 500 mg of lamivudine; and the second active ingredient consists of about 5 mg to about 8 mg of adefovir dipivoxil.

In some preferred embodiments of the invention, the first active ingredient consists of about 550 mg of lamivudine; and the second active ingredient consists of about 5 mg to about 8 mg of adefovir dipivoxil.

In some preferred embodiments of the invention, the first active ingredient consists of about 600 mg of lamivudine; and the second active ingredient consists of about 3 mg to about 5 mg of adefovir dipivoxil.

In some preferred embodiments, the pharmaceutical compositions of the invention may be in the form of solid or liquid formulations suitable for oral dosing such as tablets, capsules or solution or suspension.

In some other preferred embodiments, the pharmaceutical compositions of the invention may be in the form of injectable formulations suitable for parenteral administration.

In another aspect, the invention generally relates to the use in a method for treating hepatitis B virus infection in a human. The method includes: administering to a subject in need thereof a daily dose, in combination, of more than 400 mg to about 1500 mg of lamivudine, (2R, cis)-4-amino-1-(2-hydroxymethyl-1,3-oxathiolan-5-yl)-pyrimidin-2-one, or a therapeutically equivalent amount of a pharmaceutically acceptable salt or ester thereof, and about 1 mg to about 10 mg of adefovir dipivoxil, bis(pivaloyloxymethyl)(9-[(R)-2(phosphonomethoxy)ethyl]adenine, or a therapeutically equivalent amount of a a pharmaceutically acceptable salt or ester thereof.

In some embodiments of the invention, the method includes administering, in combination, to a subject in need thereof a daily dose of more than 400 mg to about 900 mg of lamivudine, or a therapeutically equivalent amount of a pharmaceutically acceptable salt or ester thereof and about 2 mg to about 8 mg of adefovir dipivoxil, or a therapeutically equivalent amount of a pharmaceutically acceptable salt or ester thereof.

In some embodiments of the invention, the method includes administering, in combination, to a subject in need thereof a daily dose of about 600 mg to about 900 mg of lamivudine, or a therapeutically equivalent amount of a pharmaceutically acceptable salt or ester thereof and about 2 mg to about 5 mg of adefovir dipivoxil, or a therapeutically equivalent amount of a pharmaceutically acceptable salt or ester thereof.

In some embodiments of the invention, the method includes administering, in combination, to a subject in need thereof a daily dose of about 400 mg to about 600 mg of lamivudine, or a therapeutically equivalent amount of a pharmaceutically acceptable salt or ester thereof and about 5 mg to about 8 mg of adefovir dipivoxil, or a therapeutically equivalent amount of a pharmaceutically acceptable salt or ester thereof.

In some embodiments of the invention, the method includes administering, in combination, to a subject in need thereof a daily dose of about 500 mg of lamivudine and about 5 mg to about 8 mg of adefovir dipivoxil.

In some preferred embodiments of the invention, the method includes administering, in combination, to a subject in need thereof a daily dose of about 550 mg of lamivudine and about 5 mg to about 8 mg of adefovir dipivoxil.

In some embodiments of the invention, the method includes administering, in combination, to a subject in need thereof a daily dose of about 600 mg of lamivudine and about 3 mg to about 5 mg of adefovir dipivoxil.

In some preferred embodiments of the invention, the method of administration is by way of administering a combination of the daily dose in the form of a single tablet or in the form of a single capsule.

In some other preferred embodiments, the method of administration is by way of administering a combination of the daily dose in the form of parenteral injection.

In yet another aspect, the disclosure generally relates to a method for minimizing or preventing the onset of viral resistance when treating a hepatitis B virus infection in a human with lamivudine or adefovir dipivoxil. The method includes: co-administering to the subject a daily dose of more than 400 mg to about 1500 mg of lamivudine, (2R, cis)-4-amino-1-(2-hydroxymethyl-1,3-oxathiolan-5-yl)-pyrimidin-2-one, or a therapeutically equivalent amount of a pharmaceutically acceptable salt or ester thereof, with about 1 mg to about 8 mg of adefovir dipivoxil, bis(pivaloyloxymethyl)(9-[(R)-2(phosphonomethoxy)ethyl]adenine, or a therapeutically equivalent amount of a pharmaceutically acceptable salt or ester thereof.

In some aspects, the method includes: co-administering to the subject a daily dose of about 4 mg to about 7 mg of adefovir dipivoxil, or a therapeutically equivalent amount of a pharmaceutically acceptable salt or ester thereof.

In some aspects, the method includes: co-administering to the subject a daily dose of about 3 mg to about 5 mg of adefovir dipivoxil, or a therapeutically equivalent amount of a pharmaceutically acceptable salt or ester thereof.

In yet another aspect, the disclosure generally relates to a method for rapidly restoring a patient's liver function while minimizing the risk of or preventing viral breakthrough or ALT flare. The method includes administering, in combination, to a subject in need thereof a daily dose of more than 400 mg to about 1500 mg of lamivudine, (2R, cis)-4-amino-1-(2-hydroxymethyl-1,3-oxathiolan-5-yl)-pyrimidin-2-one, or a therapeutically equivalent amount of a pharmaceutically acceptable salt or ester thereof; and about 1 mg to about 10 mg of adefovir dipivoxil, bis(pivaloyloxymethyl)(9-[(R)-2(phosphonomethoxy)ethyl]adenine, or a therapeutically equivalent amount of a pharmaceutically acceptable salt or ester thereof.

**Brief Description of the Drawings**

**FIG. 1** shows previous lamivudine dose-response curves.

**FIG. 2** shows exemplary simulated viral dynamic profiles of 4 weeks of lamivudine treatment at 5 mg to 600 mg/day.

**FIG.** 3 shows exemplary lamivudine dose optimization by $E_{max}$ modeling.

**FIG**. 4 shows an exemplary time course of serum HBV DNA in a patient following treatment initiation with *de novo* combination of lamivudine 600mg/day and adefovir dipivoxil, 5mg/day.

**FIG.** 5 shows an exemplary time course of serum ALT in a patient following treatment initiation with *die novo* combination of lamivudine 600mg/day and adefovir dipivoxil. 5mg/daym (ULN: upper limit of normal)

**Definitions**

The term "lamivudine", as used herein, refers to (2R,cis)-4- amino-1-(2-hydroxymethyl-1,3-oxathiolan-5-yl)-(1H)-pyrimidin-2-one, CAS Reg. No. 134678-17-4, having structure (I).

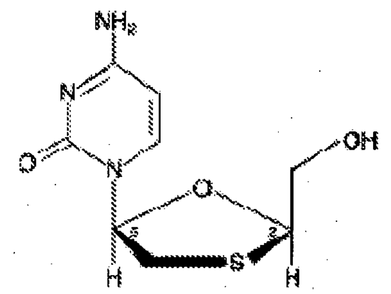

(I) Lamivudine

The term "adefovir dipivoxil", as used herein, refers to 9-[2-[[bis[(pivaloyloxy)methoxy]-phosphinyl]-methoxy]ethyl]adenine, CAS Reg. No. 142340-99-6, having structure (II)

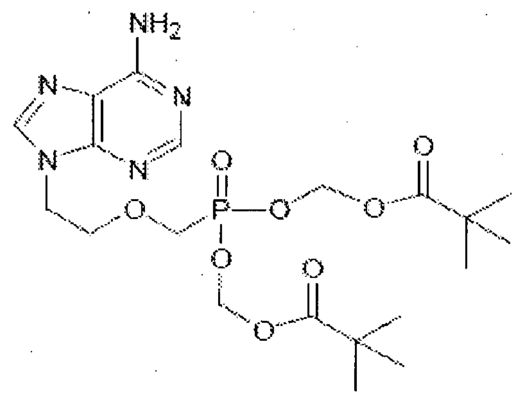

(II) Adefovir dipivoxil

As used herein, the term "pharmaceutically acceptable salt" refers to either a pharmaceutically acceptable acid addition salt or a pharmaceutically acceptable base addition salt of a currently disclosed compound that may be administered without any resultant substantial undesirable biological effect(s) or any resultant deleterious interaction(s) with any other component of a pharmaceutical composition in which it may be contained.

As used herein, the term "pharmaceutically acceptable ester," refers to esters that hydrolyze in vivo and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters include formats, acetates, propionates, butyrates, acrylates and ethylsuccinates.

**Detailed Description of the Invention**

The invention is based, in part, on the unexpected discovery of unique and synergistic combinations of lamivudine and adefovir that maximizes therapeutic effects for the treatment of hepatitis B virus infection and related conditions in humans while minimizing or preventing the development of viral resistance associated with the use of lamivudine or adefovir alone.

Lamivudine and adefovir dipivoxil are the first two small molecule oral direct HBV antivirals approved for the treatment of chronic HBV infection by the U.S. FDA in 1998 and 2002, respectively. Long-term monotherapy with lamivudine or adefovir, however, is associated with emergence of viral resistance that has ultimately limited their use as monotherapies for the treatment of chronic HBV infection. Over the past several years, a number of other nucleoside and nucleotide drugs with lower resistance rates have achieved regulatory approvals for HBV infection, including entecavir (Baraclude) and tenofovir disoproxil fumarate (Viread). Nevertheless, the low costs of lamivudine and in some areas adefovir dipivoxil make these drugs more affordable for resource-limited populations.

It is unexpectedly discovered that, when used in unique combined doses disclosed herein, lamivudine and adefovir dipivoxil, or their therapeutically equivalent and pharmaceutically acceptable salts or esters thereof, can provide synergistic clinical efficacy while minimizing or preventing viral resistance, thereby paving the way for manufacturing an effective, safe, and low-cost antiviral drug for the treatment of chronic HBV infection with a therapeutic and resistance profile similar to or better than those achieved with much more expensive, newer first-line drugs such as entecavir and tenofovir.

Cumulative clinical data have shown that, in patients with chronic hepatitis B, early viral response is critical to long-term treatment outcomes. More profound early viral suppression is associated with better-sustained viral responses with reduced rates of resistance and viral break through, as well as better overall biochemical (alanine aminotransferase or ALT normalization) and serologic (HBeAg seroconversion) responses.

In general, for a therapeutic agent, the pharmacologic effects typically increase with increasing dose and eventually reach a maximum efficacy effect. If the agent is well tolerated, it is desirable to use the agent at the dose resulting in maximum efficacy, or a lower dose resulting in near-maximum efficacy, if the maximum effect cannot be reached with an adequately tolerated, therapeutically acceptable amount of the drug.

Selection of the current clinical dose of 100 mg/day for lamivudine was largely based on the original dose-response determined in placebo-controlled randomized phase I/IIa investigations in patients with chronic HBV infection. In a key investigation, the median baseline serum HBV DNA level was 112 pg/mL (about $3.17X10^7$) or 7.5 $log_{10}$ copies/mL. Patients were treated with lamivudine 5 mg, 20 mg, 100 mg, 300 mg and 600 mg daily (QD) for 28 days. A relationship was found between the lamivudine daily dose and viral suppression (serum HBV DNA reduction). It was determined that among the lamivudine doses tested (5 mg to 600 mg/day), maximal observed viral suppression (median $\geq$ 98% from baseline) was measured at day 29 with the 100 mg/day dose and no additional benefits were seen with the 300 mg or 600 mg higher doses. A dose-response analysis using $E_{max}$ modeling of viral suppression at day 29 (% inhibition from baseline) as a function of plasma AUC (or "area under the curve") was performed. Results from the $E_{max}$ analysis confirmed that maximal viral response at day 29 was achieved with plasma exposure associated with the 100 mg/day dose of lamivudine (Johnson et al. "Clinical pharmacokinetics of lamivudine." Clin Pharmacokinet. 1999, 36(1);41-66).).

In that context, it is discovered and disclosed herein that lamivudine, at its currently approved conventional daily dose of 100 mg, has been significantly under-dosed for the treatment of chronic HBV infection. The incomplete viral suppression resulting from suboptimal systemic exposures in hepatitis B patients is associated with increased rates of resistance to lamivudine.

The above original lamivudine dose-response analysis had major limitations: (a) the HBV DNA quantitation method had a lower limit of quantitation of about 1.5 pg/mL or about $4.25X10^3$ copies/mL. With a median baseline HBV DNA at 112 pg/mL, the effective dynamic range of the assay is less than 100 fold or 2 $log_{10}$; (b) consequently the antiviral activity was expressed as percent change from baseline, and a 98% inhibition was considered as being maximal. With high daily production of about $10^{11}$-$10^{12}$ virions and pretreatment serum HBV DNA viral load typically in the order of

$10^7$-$10^9$ copies/mL in HBeAg-positive CHB patients, a 99% inhibition or 2 $log_{10}$ copies/mL. from baseline does not capture the full degree of anti-HBV clinical efficacy that is considered desirable by current expert perspectives, in which the therapeutic goal has evolved to suppression of HBV DNA to non-detectable levels, or at least below 4 $log_{10}$ copies/mL, as rapidly as possible and preferably within the first 6-12 months of treatment (Yuen, et al. "Hepatitis B virus DNA levels at week 4 of lamivudine treatment predict the 5-year ideal response" Hepatology. 2007, 46(6): 1695-703; Lok et al. "AASLD practice guidelines. Chronic hepatitis B: update 2009" Hepatology 2009, 50(3): 1-35; European Association for the Study of Liver (EASL) practice guidelines on chronic hepatitis B, 2008).

While over the years there have been data suggesting clinical benefits from using higher doses of lamivudine, few efforts have been devoted towards re-assessing the optimal doses of lamivudine for the treatment of chronic hepatitis B. The results of the reported studies of this type have however failed to discover a compelling benefit for higher doses of lamivudine. (Wang et al. "Kinetics of hepatitis B viral load during 48 weeks of treatment with 600 mg vs. 100 mg of lamivudine daily" J. Viral Hepat. 2004, 11(5):443-7; Torre et al. "Initial high dose of lamivudine delays the appearance of viral resistance in chronic hepatitis B patients" Hepatology 2009, 50(4, suppl.):A524-A525.)

Herein disclosed, discovered through unique viral dynamic analyses followed by $E_{max}$ modeling, are the optimal doses of lamivudine for the treatment of chronic hepatitis B to be in the range of more than 400 mg/day to about 1500 mg/day, substantially greater than the regulatory-approved and guideline-recommended approved dose of 100 mg/day.

Greatly improved early and sustained viral responses with the optimal doses of lamivudine are expected to minimize viral resistance to lamivudine. However, increasing the lamivudine dose, alone, may not be sufficient enough to completely block viral resistance; for example, one patient treated with 600 mg lamivudine once daily developed viral resistance in an early trial (Wang et al. "Kinetics of hepatitis B viral load during 48 weeks of treatment with 600 mg vs. 100 mg of lamivudine daily" J. Viral Hepat. 2004, 11(5):443-7.)

HBV mutants resistant to lamivudine remain susceptible to adefovir and vice versa. *In vitro* data have shown enhanced antiviral activity between lamivudine and adefovir. (Shaw et al. "Synergistic inhibition in vitro of hepadnaviral replication by PMEA and penciclovir or lamivudine" Antiviral Res. 1997, 34(2):A51; Colledge et al. "In vitro antihepadnaviral activities of combinations of penciclovir, lamivudine, and adefovir" Antimicrob Agents Chemother. 2000, 44(3):551-560; Delaney et al. "Combinations of adefovir with nucleoside analogs produce additive antiviral effects against hepatitis B virus in vitro" Antimicrob Agents Chemother, 2004, 48(10):3702-3710.) However, as noted above, when used as a *de novo* combination treatment at regulatory-approved clinical doses in patients who were naïve to nucleoside/nucleotide therapy, no synergistic or additive antiviral activity was observed. (Lok et al. "AASLD practice guidelines. Chronic hepatitis B: update 2009" Hepatology 2009, 50(3): 1 -35.).

Also, at their respective regulatory-approved clinical doses, *de novo* combination therapy with lamivudine and adefovir in treatment-naïve patients failed to adequately block lamivudine resistance. One prospectively designed clinical study included 115 patients randomized to receive lamivudine alone or lamivudine in combination with adefovir dipivoxil. At week 52, there was no significant difference in HBV DNA reduction, ALT normalization or HBeAg loss. At week 52 and week 104, the rate of genetically-detectable viral resistance in the combination group was still substantial, i.e. 9% at week 52 and 15% at week 104 (Sung et al. "Lamivudine compared with lamivudine and adefovir dipivoxil for the treatment of HBeAg-positive chronic hepatitis B" J. Hepatol. 2008, 48(5):728-735).

Currently, due to lack of compelling data, the *de novo* combination of lamivudine and adefovir dipivoxil is not recommended for therapeutic use by clinical experts, as noted in several treatment guidelines (AASLD practice guidelines, Guideline on prevention and treatment of chronic hepatitis B in China 2005).

We found that *de novo* combination of lamivudine and adefovir would not optimally minimize or prevent viral resistance to lamivudine unless lamivudine is used at optimal doses of more than 400 mg/day to about 1500 mg/day, which we discovered through unique viral dynamic analyses and $E_{max}$ modeling.

Since the newly-discovered optimal doses of lamivudine disclosed herein are expected to produce more profound early and sustained viral suppression and reduce the rate of viral resistance through more effective suppression of HBV replication, the role of adefovir in the combination is primarily to prevent/suppress the emergence of any lamivudine-resistant HBV variants, as adefovir itself has intrinsically modest anti-HBV efficacy and higher doses of adefovir are associated with nephrotoxicity.

Furthermore, with the optimal doses of lamivudine disclosed herein that produce greater early and sustained viral suppression leading to lower residual viral load, the dose of adefovir dipivoxil required for preventing/suppressing lamivudine resistance in the combination can in fact be reduced for potentially improved long-term safety. Such a reduced daily dose of adefovir dipivoxil to less than 10 mg is desirable given the 3% cumulative rate of nephrotoxicity associated with long-term adefovir therapy.

As people skilled in the art would appreciate, unlike human immunodeficiency virus, there is currently no cell propagation assay that would allow for *in vitro* selection of resistant hepatitis B viral mutants against lamivudine, adefovir, or any other nucleoside/nucleotide analogs. Therefore, the ability of adefovir at concentrations associated with doses less than 10 mg/day to minimize or to prevent emergence of resistance to lamivudine at concentrations of lamivudine associated with its optimal doses cannot be assessed *in vitro*.

The only way to provide direct supportive data would be to conduct a clinical trial in treatment-naïve patients who receive *de novo* combination of lamivudine and adefovir dipivoxil at optimal doses.

In one aspect, the invention generally relates to a pharmaceutical composition for treating hepatitis B infection virus infection in a human. The pharmaceutical composition includes: a first active pharmaceutical ingredient consisting of more than 400 mg (e.g., 410 mg, 450 mg, 480 mg, 500 mg) to about 1,500 mg of lamivudine, (2R, cis)-4-amino-1-(2-hydroxymethyl-1,3-oxathiolan-5-yl)-pyrimidin-2-one, or a therapeutically equivalent amount of pharmaceutically acceptable salt or ester thereof; and a second active pharmaceutical ingredient consisting of about 1 mg to about 10 mg of adefovir dipivoxil, bis(pivaloyloxymethyl)(9-[(R)-2(phosphonomethoxy)ethyl]adenine, or a therapeutically equivalent amount of pharmaceutically acceptable salt or ester thereof.

In some embodiments of the invention, the first active ingredient consists of about 600 mg to about 900 mg of lamivudine, or a therapeutically equivalent amount of pharmaceutically acceptable salt or ester thereof; and the second active ingredient consists of about 2 mg to about 5 mg of adefovir dipivoxil, or a therapeutically equivalent amount of pharmaceutically acceptable salt or ester thereof.

In some preferred embodiments of the invention, the first active ingredient consists of about 400 mg to about 600 mg of lamivudine, or a therapeutically equivalent amount of pharmaceutically acceptable salt or ester thereof; and the second active ingredient consists of about 5 mg to about 8 mg of adefovir dipivoxil, or a therapeutically equivalent amount of pharmaceutically acceptable salt or ester thereof.

In some preferred embodiments of the invention, the first active ingredient consists of about 450 mg to about 650 mg of lamivudine, or a therapeutically equivalent amount of pharmaceutically acceptable salt or ester thereof; and the second active ingredient consists of about 3 mg to about 7 mg of adefovir dipivoxil, or a therapeutically equivalent amount of pharmaceutically acceptable salt or ester thereof.

In some preferred embodiments of the invention, the first active ingredient consists of about 500 mg of lamivudine; and the second active ingredient consists of about 5 mg to about 8 mg of adefovir dipivoxil.

In some preferred embodiments of the invention, the first active ingredient consists of about 500 mg of lamivudine; and the second active ingredient consists of about 5 mg (or about 5.5 mg) of adefovir dipivoxil.

In some preferred embodiments of the invention, the first active ingredient consists of about 500 mg of lamivudine; and the second active ingredient consists of about 6 mg (or about 6.5 mg) of adefovir dipivoxil.

In some preferred embodiments of the invention, the first active ingredient consists of about 500 mg of lamivudine; and the second active ingredient consists of about 7 mg (or about 7.5 mg) of adefovir dipivoxil.

In some preferred embodiments of the invention, the first active ingredient consists of about 550 mg of lamivudine; and the second active ingredient consists of about 8 mg of adefovir dipivoxil.

In some preferred embodiments of the invention, the first active ingredient consists of about 550 mg of lamivudine; and the second active ingredient consists of about 5 mg to about 8 mg of adefovir dipivoxil.

In some preferred embodiments of the invention, the first active ingredient consists of about 550 mg of lamivudine; and the second active ingredient consists of about 5 mg (or about 5.5 mg) of adefovir dipivoxil.

In some preferred embodiments of the invention, the first active ingredient consists of about 550 mg of lamivudine; and the second active ingredient consists of about 6 mg (or about 6.5 mg) of adefovir dipivoxil.

In some preferred embodiments of the invention, the first active ingredient consists of about 550 mg of lamivudine; and the second active ingredient consists of about 7 mg (or about 7.5 mg) of adefovir dipivoxil.

In some preferred embodiments of the invention, the first active ingredient consists of about 550 mg of lamivudine; and the second active ingredient consists of about 8 mg of adefovir dipivoxil.

In some preferred embodiments of the invention, the first active ingredient consists of about 600 mg of lamivudine; and the second active ingredient consists of about 3 mg to about 5 mg of adefovir dipivoxil.

In some preferred embodiments of the invention, the first active ingredient consists of about 600 mg of lamivudine; and the second active ingredient consists of about 3 mg (or about 3.5 mg) of adefovir dipivoxil.

In some preferred embodiments of the invention, the first active ingredient consists of about 600 mg of lamivudine; and the second active ingredient consists of about 4 mg (or about 4.5 mg) of adefovir dipivoxil.

In some preferred embodiments, the pharmaceutical compositions of the invention may be in the form of solid or liquid formulations suitable for oral dosing such as tablets, capsules or solution or suspension.

In some other preferred embodiments, the pharmaceutical compositions of the invention may be in the form of injectable formulations suitable for parenteral administration.

In another aspect, the invention generally relates to a method for treating hepatitis B virus infection in a human. The method includes: administering, in combination, to a subject in need thereof a daily dose of more than 400 mg to about 1500 mg of lamivudine, (2R, cis)-4-amino-1-(2-hydroxymethyl-1,3-oxathiolan-5-yl)-pyrimidin-2-one, or a therapeutically equivalent amount of pharmaceutically acceptable salt or ester thereof, and about 1 mg to about 10 mg of adefovir dipivoxil, bis(pivaloyloxymethyl)(9-[(R)-2(phosphonomethoxy)ethyl]adenine, or a therapeutically equivalent amount of pharmaceutically acceptable salt or ester thereof.

In some embodiments of the invention, the method includes administering, in combination, to a subject in need

thereof a daily dose of more than 400 mg to about 900 mg of lamivudine, or a therapeutically equivalent amount of pharmaceutically acceptable salt or ester thereof and about 2 mg to about 8 mg of adefovir dipivoxil, or a therapeutically equivalent amount of pharmaceutically acceptable salt or ester thereof.

In some preferred embodiments of the invention, the method includes administering, in combination, to a subject in need thereof a daily dose of more than 400 mg to about 600 mg of lamivudine, or a therapeutically equivalent amount of pharmaceutically acceptable salt or ester thereof and about 5 mg to about 8 mg of adefovir dipivoxil, or a therapeutically equivalent amount of pharmaceutically acceptable salt or ester thereof.

In some preferred embodiments of the invention, the method includes administering, in combination, to a subject in need thereof a daily dose of about 600 mg to about 900 mg of lamivudine, or a therapeutically equivalent amount of pharmaceutically acceptable salt or ester thereof and about 2 mg to about 5 mg of adefovir dipivoxil, or a therapeutically equivalent amount of pharmaceutically acceptable salt or ester thereof.

In some preferred embodiments of the invention, the method includes administering, in combination, to a subject in need thereof a daily dose of about 450 mg to about 650 mg of lamivudine, or a therapeutically equivalent amount of pharmaceutically acceptable salt or ester thereof and about 3 mg to about 7 mg of adefovir dipivoxil, or a therapeutically equivalent amount of pharmaceutically acceptable salt or ester thereof.

In some preferred embodiments of the invention, the method includes administering, in combination, to a subject in need thereof a daily dose of about 500 mg of lamivudine and about 5 mg to about 8 mg of adefovir dipivoxil.

In some preferred embodiments of the invention, the method includes administering, in combination, to a subject in need thereof a daily dose of about 500 mg of lamivudine and about 5 mg (or about 5.5 mg) of adefovir dipivoxil.

In some preferred embodiments of the invention, the method includes administering, in combination, to a subject in need thereof a daily dose of about 500 mg of lamivudine and about 6 mg (or about 6.5 mg) of adefovir dipivoxil.

In some preferred embodiments of the invention, the method includes administering, in combination, to a subject in need thereof a daily dose of about 500 mg of lamivudine and about 7 mg (or about 7.5 mg) of adefovir dipivoxil.

In some preferred embodiments of the invention, the first active ingredient consists of about 550 mg of lamivudine; and the second active ingredient consists of about 8 mg of adefovir dipivoxil.

In some preferred embodiments of the invention, the method includes administering, in combination, to a subject in need thereof a daily dose of about 550 mg of lamivudine; and about 5 mg to about 8 mg of adefovir dipivoxil.

In some preferred embodiments of the invention, the method includes administering, in combination, to a subject in need thereof a daily dose of about 550 mg of lamivudine and about 5 mg (or about 5.5 mg) of adefovir dipivoxil.

In some preferred embodiments of the invention, the method includes administering, in combination, to a subject in need thereof a daily dose of about 550 mg of lamivudine and about 6 mg (or about 6.5 mg) of adefovir dipivoxil.

In some preferred embodiments of the invention, the method includes administering, in combination, to a subject in need thereof a daily dose of about 550 mg of lamivudine and about 7 mg (or about 7.5 mg) of adefovir dipivoxil.

In some preferred embodiments of the invention, the method includes administering, in combination, to a subject in need thereof a daily dose of about 550 mg of lamivudine and about 8 mg of adefovir dipivoxil.

In some preferred embodiments of the invention, the method includes administering, in combination, to a subject in need thereof a daily dose of about 600 mg of lamivudine; and about 3 mg to about 5 mg of adefovir dipivoxil.

In some preferred embodiments of the invention, the method includes administering, in combination, to a subject in need thereof a daily dose of about 600 mg of lamivudine and about 3 mg (or about 3.5 mg) of adefovir dipivoxil.

In some preferred embodiments of the invention, the method includes administering, in combination, to a subject in need thereof a daily dose of about 600 mg of lamivudine and about 4 mg (or about 4.5 mg) of adefovir dipivoxil.

In some preferred embodiments of the invention, the method includes administering, in combination, to a subject in need thereof a daily dose of about 600 mg of lamivudine and about 5 mg (or about 5.5 mg) of adefovir dipivoxil.

In some preferred embodiments of the invention, the method includes administering, in combination, to a subject in need thereof a daily dose of about 650 mg of lamivudine; and about 2 mg to about 5 mg of adefovir dipivoxil.

In some preferred embodiments of the invention, the method includes administering, in combination, to a subject in need thereof a daily dose of about 650 mg of lamivudine and about 3 mg (or about 3.5 mg) of adefovir dipivoxil.

In some preferred embodiments of the invention, the method includes administering, in combination, to a subject in need thereof a daily dose of about 650 mg of lamivudine and about 4 mg (or about 4.5 mg) of adefovir dipivoxil.

In some preferred embodiments of the invention, the method includes administering, in combination, to a subject in need thereof a daily dose of about 650 mg of lamivudine and about 5 mg (or about 5.5 mg) of adefovir dipivoxil.

In some preferred embodiments of the invention, the method of administration is by way of administering a combination of the daily dose in the form of a single tablet.

In some preferred embodiments of the invention, the method of administration is by way of administering a combination of the daily dose in the form of a single capsule.

In some preferred embodiments of the invention, the method of administration is by way of administering a combination of the daily dose in the form of a solution or suspension.

In some other preferred embodiments of the invention, the method of administration is by way of administering

a combination of the daily dose in the form of an injectable solution.

In yet another aspect, the invention generally relates to a method for minimizing or preventing viral resistance when treating hepatitis B virus infection in a human with lamivudine. The method includes: co-administering to the subject a daily dose of more than 400 mg to about 1500 mg of lamivudine, (2R, cis)-4-amino-1-(2-hydroxymethyl-1,3-oxathiolan-5-yl)-pyrimidin-2-one, or a therapeutically equivalent amount of a pharmaceutically acceptable salt or ester thereof, with a daily dose of about 1 mg to about 8 mg, of adefovir dipivoxil, bis(pivaloyloxymethyl)(9-[(R)-2(phosphonomethoxy)ethyl]adenine, or a therapeutically equivalent amount of pharmaceutically acceptable salt or ester thereof.

In some embodiments of the invention, the method includes: co-administering to the subject a daily dose of about 4 mg to about 7 mg of adefovir dipivoxil, or a therapeutically equivalent amount of a pharmaceutically acceptable salt or ester thereof.

In some embodiments of the invention, the method includes: co-administering to the subject a daily dose of about 3 mg to about 5 mg of adefovir dipivoxil, or a therapeutically equivalent amount of pharmaceutically acceptable salt or ester thereof.

In some embodiments of the invention, the method includes: co-administering to the subject a daily dose of about 3 mg (or about 3.5 mg) of adefovir dipivoxil, or a therapeutically equivalent amount of pharmaceutically acceptable salt or ester thereof.

In some embodiments of the invention, the method includes: co-administering to the subject a daily dose of about 4 mg (or about 4.5 mg) of adefovir dipivoxil, or a therapeutically equivalent amount of pharmaceutically acceptable salt or ester thereof.

In some embodiments of the invention, the method includes: co-administering to the subject a daily dose of about 5 mg (or about 5.5 mg) of adefovir dipivoxil, or a therapeutically equivalent amount of pharmaceutically acceptable salt or ester thereof.

In some embodiments of the invention, the method includes: co-administering to the subject a daily dose of about 6 mg (or about 6.5 mg) of adefovir dipivoxil, or a therapeutically equivalent amount of pharmaceutically acceptable salt or ester thereof.

In some embodiments of the invention, the method includes: co-administering to the subject a daily dose of about 7 mg (or about 7.5 mg) of adefovir dipivoxil, or a therapeutically equivalent amount of pharmaceutically acceptable salt or ester thereof.

In yet another aspect, the invention generally relates to a method for rapidly restoring a patient's liver function while minimizing the risk of or preventing viral breakthrough or ALT flare. The method includes administering, in combination, to a subject in need thereof a daily dose of more than 400 mg to about 1500 mg of lamivudine, (2R, cis)-4-amino-1-(2-hydroxymethyl-1,3-oxathiolan-5-yl)-pyrimidin-2-one, or a therapeutically equivalent amount of a pharmaceutically acceptable salt or ester thereof; and about 1 mg to about 10 mg of adefovir dipivoxil, bis(pivaloyloxymethyl)(9-[(R)-2(phosphonomethoxy)ethyl]adenine, or a therapeutically equivalent amount of a pharmaceutically acceptable salt or ester thereof.

The time course of ALT normalization typically parallels that of HBV DNA decline. Thus, with an enhanced early and more persistent viral suppression by combination of optimal doses of lamivudine with reduced doses of adefovir that leads to undetectable viral level faster than regular doses of lamivudine and adefovir, ALT normalization will also occur sooner following a much faster time course. Viral breakthrough, defined as an abrupt increase in serum HBV DNA levels following persistent suppression, may occur due to non-compliance but more often with the emergence of resistant mutants, and is typically accompanied by ALT flare defined as an ALT increase to > 2X upper limit of the normal range.

Compounds that are basic in nature are generally capable of forming a wide variety of different salts with various inorganic and/or organic acids. Although such salts are generally pharmaceutically acceptable for administration to animals and humans, it may be desirable in practice to initially isolate a compound from the reaction mixture as a pharmaceutically unacceptable salt and then simply convert the latter back to the free base compound by treatment with an alkaline reagent, and subsequently convert the free base to a pharmaceutically acceptable acid addition salt. The acid addition salts of the base compounds can be readily prepared using conventional techniques, e.g., by treating the base compound with a substantially equivalent amount of the chosen mineral or organic acid in an aqueous solvent medium or in a suitable organic solvent such as, for example, methanol or ethanol. Upon careful evaporation of the solvent, the desired solid salt is obtained.

Acids which can be used to prepare the pharmaceutically acceptable acid-addition salts of the base compounds are those which can form non-toxic acid-addition salts, e.g., salts containing pharmacologically acceptable anions, such as chloride, bromide, iodide, nitrate, sulfate or bisulfate, phosphate or acid phosphate, acetate, lactate, citrate or acid citrate, tartrate or bitartrate, succinate, maleate, fumarate, gluconate, saccharate, benzoate, methanesulfonate and pamoate [i.e., 1,1'-methylene-bis-(2-hydroxy-3-naphthoate) salts.

Examples of suitable acids for lamivudine include hydrochloric, hydrobromic, sulphuric, nitric, perchloric, fumaric, maleic, phosphoric, glycollic, lactic, salicylic, succinic, toluene-p-sulphonic, tartaric, acetic, citric, methanesulphonic, formic, benzoic, malonic, naphtalene-2-sulphonic and benzenesulphonic acids.

Compounds that are acidic in nature, e.g., contain a COOH or tetrazole moiety, are generally capable of forming a wide variety of salts with various inorganic and/or organic bases. Although such salts are generally pharmaceutically acceptable for administration to animals and humans, it may be desirable in practice to initially isolate a compound from the reaction mixture as a pharmaceutically unacceptable salt and then simply convert the latter back to the free acid compound by treatment with an acidic reagent, and subsequently convert the free acid to a pharmaceutically acceptable base addition salt. These base addition salts can be readily prepared using conventional techniques, e.g., by treating the corresponding acidic compounds with an aqueous solution containing the desired pharmacologically acceptable cations, and then evaporating the resulting solution to dryness, preferably under reduced pressure. Alternatively, they also can be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum product yields of the desired solid salt.

Bases which can be used to prepare the pharmaceutically acceptable base-addition salts of the base compounds are those which can form non-toxic base-addition salts, e.g., salts containing pharmacologically acceptable cations, such as, alkali metal cations (e.g., potassium and sodium), alkaline earth metal cations (e.g., calcium and magnesium), ammonium or other watersoluble amine addition salts such as N-methylglucamine-(meglumine), lower alkanolammonium and other such bases of organic amines.

Pharmaceutically acceptable esters of lamivudine include, for example, (1) carboxylic acid esters in which the non-carbonyl moiety of the carboxylic acid portion of the ester grouping is selected from straight or branched chain alkyl (for example, methyl, n-propyl, t-butyl, or n-butyl), cycloalkyl, alkoxyalkyl (for example, methoxymethyl), aralkyl (for example, benzyl), aryloxyalkyl (for example, phenoxymethyl), aryl (for example, phenyl optionally substituted by, for example, halogen, $C_{1-4}$ alkyl, or $C_{1-4}$ alkoxy), or amino; (2) sulphonate esters, such as alkyl- or aralkylsulphonyl (for example, methanesulphonyl); (3) amino acid esters (for example, L-valyl or L-isoleucyl); and (4) phosphonate esters. In such esters, unless otherwise specified, any alkyl moiety present advantageously contains from 1 to 12 carbon atoms, particularly from 1 to 6 carbon atoms, more particularly from 1 to 3 carbon atoms. Any cycloalkyl moiety present in such esters may contain from 2 to 6 carbon atoms. Any aryl moiety present in such esters may comprise a phenyl group. Any reference to any of the above compounds also includes a reference to a physiologically acceptable salt thereof.

Presently disclosed pharmaceutical compositions can be used in an animal or human. Thus, a presently disclosed compound can be formulated as a pharmaceutical composition for oral, buccal, parenteral (e.g., intravenous, intramuscular or subcutaneous), topical, rectal or intranasal administration or in a form suitable for administration by inhalation or insufflation. The pharmaceutical compositions may be in unit dosage form and may be prepared by any methods well known in the art.

The active ingredients may be formulated with a carrier which constitutes one or more accessory ingredients. In general, the formulations are prepared by uniformly and intimately bringing into association the active ingredients with liquid carriers or finely divided solid carriers or both, and then if necessary shaping the product. The pharmaceutically acceptable carrier can be any such carrier known in the art including those described in, for example, Remington's Pharmaceutical Sciences, Mack Publishing Co., (A. R. Gennaro edit. 1985). Pharmaceutical compositions of the compounds presently disclosed may be prepared by methods known in the art including, for example, mixing at least one presently disclosed compound with a pharmaceutically acceptable carrier.

For oral administration, the pharmaceutical composition may take the form of, for example, a tablet or capsule prepared by conventional methods with a pharmaceutically acceptable excipient(s) such as a binding agent (e.g., pregelatinized maize starch, polyvinylpyrrolidone or hydroxypropyl methylcellulose); filler (e.g., lactose, microcrystalline cellulose or calcium phosphate); lubricant (e.g., magnesium stearate, talc or silica); disintegrant (e.g., potato starch or sodium starch glycolate); and/or wetting agent (e.g., sodium laurel sulphate). The tablets may be coated by methods known in the art. Liquid preparations for oral administration may take the form of a, for example, solution, syrup or suspension, or they may be presented as a dry product for constitution with water or other suitable vehicle before use. Such liquid preparations may be prepared by conventional methods with a pharmaceutically acceptable additive(s) such as a suspending agent (e.g., sorbitol syrup, methyl cellulose or hydrogenated edible fats); emulsifying agent (e.g., lecithin or acacia); non-aqueous vehicle (e.g., almond oil, oily esters or ethyl alcohol); and/or preservative (e.g., methyl or propyl p-hydroxybenzoates or sorbic acid).

For buccal administration, the composition may take the form of tablets or lozenges formulated in a conventional manner.

Presently disclosed compounds may be formulated for parenteral administration by injection, including using conventional catheterization techniques or infusion. Formulations for injection may be presented in unit dosage form, e.g., in ampules or in multi-dose containers, with an added preservative. The compositions may take such forms as suspensions, solutions or emulsions in oily or aqueous vehicles, and may contain a formulating agent such as a suspending, stabilizing and/or dispersing agent recognized by those of skill in the art. Alternatively, the active ingredient may be in powder form for reconstitution with a suitable vehicle, e.g., sterile pyrogen-free water, before use.

For topical administration, a presently disclosed compound may be formulated as an ointment or cream.

Presently disclosed compounds may also be formulated in rectal compositions such as suppositories or retention enemas, e.g., containing conventional suppository bases such as cocoa butter or other glycerides.

For intranasal administration or administration by inhalation, presently disclosed compounds may be conveniently delivered in the form of a solution or suspension from a pump spray container that is squeezed or pumped by the patient or as an aerosol spray presentation from a pressurized container or a nebulizer, with the use of a suitable propellant, e.g., dlchlorodifluoromethane, trichlorofluoromethane, dichlorotekrafluoroethane, carbon dioxide or other suitable gas. In the case of a pressurized aerosol, the dosage unit may be determined by providing a valve to deliver a nietered amount. The pressurized container or nebulizer may contain a solution or suspension. of the presently disclosed compound. Capsules and cartridges (made, for example, from gelatin) for use in an inhaler or insufflator may be formulated containing a powder mix of a presently disclosed compound and a suitable powder base such as lactose or starch. Preferred unit dosage formulations are those containing a daily dose or daily subdose of the active ingredients.

A tablet may be made by compression or molding, optionally with one or more accessory ingredients. Compressed tablets may be prepared by compressing in a suitable machine the active ingredients in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose) surface-active or dispersing agent. Molded tablets may be made by molding a mixture of the powdered compound moistened with an inert liquid diluent in a suitable machine. The tablets may optionally be coated or scored and may be formulated so as to provide slow or controlled release of the active ingredients therein using, for example, hydroxypropylmethyl cellulose in varying proportions to provide the desired release profile. Tablets may optionally be provided with an enteric coating, to provide release in parts of the gut other than the stomach.

The compounds presently disclosed may also be formulated for sustained delivery according to methods well known to those of ordinary skill in the art. Examples of such formulations can be found in United States Patents 3,119,742; 3,492,397; 3,538,214; 4,060,598; and 4,173,626.

The pharmaceutical compositions of the invention may include other agents and/or components. For example, those suitable for oral administration may include such further agents as sweeteners, thickeners and flavoring compounds; and/or such further components as a physical barrier within a dosage unit that separates lamivudine from adefovir for better stability/dissolution profiles as determined by their respective physiochemical properties.

The following examples are intended for illustration purposes only.

## Examples

*Example 1. Formulations*

While lamivudine and adefovir dipivoxil may be co-administered as raw chemicals, it is preferable to deliver these compounds in a pharmaceutical composition suitable for administration to patients. Exemplary amounts of lamivudine and adefovir dipivoxil for the preparation of pharmaceutical compositions is indicated in **Table 1.**

**Table 1.** Exemplary unit doses of lamivudine and adefovir dipivoxil for the preparation of pharmaceutical compositions

| | | **Lamivudine** (mg/unit dose) | | | | | |
|---|---|---|---|---|---|---|---|
| | | **405** | **460** | **510** | **550** | **600** | **900** |
| **Adefovir dipivoxil** (mg/unit dose) | **3** | 405/3 | 460/3 | 510/3 | 550/3 | 600/3 | 900/3 |
| | **5** | 405/5 | 460/5 | 510/5 | 550/5 | 600/5 | 900/5 |
| | **8** | 405/8 | 460/8 | 510/8 | 550/8 | 600/8 | 900/8 |
| | **10** | 405/10 | 460/10 | 510/10 | 550/10 | 600/10 | 900/10 |

Each of the exemplary lamivudine dose amounts, i.e., 405, 460, 510, 550, 600 and 900 mg per unit dose, is co-formulated with each of the exemplary adefovir dipivoxil dose amounts, i.e., 3, 5, 8 and 10 mg per unit dose, resulting in 24 strengths of the prepared compositions.

Each of the strengths in **Table 1** is formulated with appropriate pharmaceutical excipients into common pharmaceutical preparations intended to be given by routes including but not limited to oral, parenteral, topical, rectal, nasal or vaginal administration. Considering the chronic nature of hepatitis B infection requiring long term therapy, oral formulations as tablet (caplet, pill), capsule (hard or soft shell) and liquid (solution, suspension, or emulsion) are generally preferred while parenteral formulations are useful alternatives under special situations where oral administration cannot be performed. For this reason, examples herein are only given for the most common oral and parenteral formulations.

**Table 2.1** and **Table 2.2** below present exemplary excipients and their amounts per unit dose for the co-formulation of lamivudine and adefovir dipivoxil according to the strengths listed in **Table 1.** The total weight of a unit dose is the sum of the weight of active ingredients **(Table 1)** and that of excipients **(Table 2.1** and **Table 2.2).**

**Table 2.1.** Exemplary oral formulations

| **Tablet or Capsule** | | | **Solution** | |
|---|---|---|---|---|
| Lamivudin | (Table 1) | | Lamivudine | (Table 1) |
| Adefovir dipivoxil | (Table 1) | | Adefovir dipivoxil | (Table 1) |
| Microcrystalline cellulose | 70 mg | | Sodium citrate | 0.3 g |
| Povidone | 10 mg | | Sodium bezonate | 0.05 g |
| Sodium starch glycolate | 10 mg | | Raspberry flavor | 0.1 mL |
| Magnesium stearate | 5 mg | | Purified water q.s. to | 20 mL |

**Table 2.2.** Exemplary parenteral formulations

| **Intravenous** | | | **Intramuscular** | |
|---|---|---|---|---|
| Lamivudin | (Table 1) | | Lamivudine | (Table 1) |
| Adefovir dipivoxil | (Table 1) | | Adefovir dipivoxil | (Table 1) |
| Sterile, pyrogen-free, D5 saline pH 7.0 , q.s. to | 20 mL | | Benzyl alcohol | 0.08 g |
| | | | Propylene glycol | 0.9 g |
| | | | Injectable saline q.s. to | 4.0 mL |

*Example 2. Viral dynamic analyses and $E_{max}$ model*

HBV DNA data

The HBV DNA data used in the unique assessments disclosed herein of HBV viral dynamics under lamivudine therapy were obtained from the previously published lamivudine dose-response curves **(FIG. 1).** (Johnson et al. "clinical pharmacokinetics of lamivudine" Clin. Pharmacokinet. 1999, 36(1):41-66.) Data points up to day 29 in **FIG.** 1 were digitalized. Since data on day 22 and 29 for the high doses were based on HBV DNA values that were too close to the lower limit of quantitation of the assay, those data were not considered as reliable. Therefore, for the viral dynamic analyses, only data up to day 15 were used.

Viral dynamic analyses

The digitalized data were converted to percent change from baseline as $(V_t-V_0)/V_0$ and further presented as $V_t/V_0$, where $V_0$ and $V_t$ are the HBV DNA data at baseline and at time t after treatment initiation, respectively. The viral dynamic model as illustrated below was fitted to the $V_t/V_0$ vs. time data:

$$V_t/V_0 = [(\varepsilon*u-a)/(u-a)]*\exp(-u*t) + [(1-\varepsilon)*u/(u-a)]*\exp(-a*t)$$

where u is the clearance rate constant of free virions; a is the clearance rate constant of infected cells; ε is the inhibition efficacy. (Tsiang et al. "Biphasic clearance kinetics of hepatitis B virus from patients during adefovir dipivoxil therapy," Hepatology 1999, 29(6):1863-1869.) Since the half-life of free HBV virions has been determined to be about 1 day, the parameter u was therefore fixed at 0.693/1=0.7 1/day.

The model estimates of viral dynamic parameters are presented in **Table 3** below.

**Table 3.** Exemplary viral dynamic parameter estimates (standard error) of lamivudine

| VD Parameters | **Lamivudine** doses (mg/day) | | | | |
|---|---|---|---|---|---|
| | **5** | **20** | **100** | **300** | **600** |
| **a (1/day)** | 0.0017 (0.0107) | 0.0557 (0.0299) | 0.1095 (0.0179) | 0.1280 (0.0121) | 0.1880 (0.0278) |
| **e** | 0.6193 (0.0369) | 0.8186 (0.0374) | 0.8173 (0.0254) | 0.8463 (0.0087) | 0.8673 (0.0248) |

The model estimates were then used to simulate viral dynamic profiles for the 5 doses of lamivudine for the purpose of (a) showing goodness-of-fit; and (b) predicting viral response beyond day 15. These analyses are illustrated in **FIG. 2.**

**FIG. 2** shows a close agreement between experimental data and simulated curves, demonstrating an excellent goodness-of-fit. The predicted viral response as reduction from baseline ($log_{10}$ scale) on days 8, 15, 22 and 29 are presented in **Table 4** below.

**Table 4.** Examples of model predicted HBV DNA reduction from baseline on $long_{10}$ scale (% change)

| Day | **Lamivudine** doses (mg/day) | | | | |
|---|---|---|---|---|---|
| | **5** | **20** | **100** | **300** | **600** |
| **8** | 0.42 (61.1) | 0.89 (87.1) | 1.03 (90.7) | 1.15 (92.9) | 1.36 (95.7) |
| **15** | 0.43 (62.8) | 1.07 (91.5) | 1.38 (95.8) | 1.56 (97.2) | 1.97 (98.9) |
| **22** | 0.43 (63.2) | 1.24 (94.2) | 1.71 (98.1) | 1.95 (98.9) | 2.54 (99.7) |
| **29** | 0.44 (63.7) | 1.41 (96.1) | 2.04 (99.1) | 2.34 (99.5) | 3.11 (99.9) |

Viral response (>2 $log_{10}$) beyond the dynamic range of the original assay was successfully predicted by our unique viral dynamic analyses. These results demonstrated a clear efficacy benefit of higher doses of lamivudine: as shown in **Table 4,** the 600 mg doses achieved 1 $log_{10}$ or 10 times more reduction in viral load than the 100 mg dose. Such substantial gain in early viral response, shown here to be achievable with high doses of lamivudine, has been shown to predict higher rates ofHBV DNA clearance to non-detectable levels and lower rates of resistance, thereby achieving substantial improvements in patients' efficacy outcomes. (Wang et al. "Kinetics of hepatitis B viral load during 48 weeks of treatment with 600 mg vs 100 mg of lamivudine daily" J. Viral Hepat. 2004, 11(5):443-7.)

$E_{max}$ modeling

The predicted HBV DNA reduction data on day 29 were used for $E_{max}$ modeling analyses for the purpose of defining optimal doses of lamivudine.

An $E_{max}$ model in the form of

$$E=E_{max}*ED_{50}^{h}/(D^{h}+ED_{50}^{h})$$

where E and $E_{max}$ are the viral response with dose D and maximum viral response respectively; $ED_{50}$ is the dose resulting in 50% of viral response; h is the Hill parameter. The maximum reduction of HBV NDA at 4 weeks achievable by a nucleoside/nucleotide antiviral is approximately 4 $log_{10}$. (Buti et al. J. Hepatol. 39: S139-S142, 2003.) Therefore, in the $E_{max}$ modeling herein, $E_{max}$ was fixed to 4.

The $E_{max}$ model estimates (standard error) for $ED_{50}$ and h are 101.5 (29.04) mg and 0.6085 (0.0937), respectively. Using these estimates, viral responses at untested doses were predicted and results are illustrated in **FIG. 3** and presented **in Table 5.**

The $E_{max}$ results indicated that the regulatory approved clinical dose or lamivudine would produce about 2 $log_{10}$ reduction from baseline or only about 1/100 of the maximum potentially achievable effect one month after therapy. By contrast, as shown in **FIG.** 3 and **Table 5,** doses > 400 mg/day are predicted to produce greater antiviral effects of about 2.8-3.3 $log_{10}$ with 400 mg/day to 1500 mg/day. Considering the incremental antiviral effect by higher doses from the 100 mg dose as well as cost of goods, doses of more than 400 mg/day to about 900 mg/day are considered as optimal. These doses are predicted to produce about 10-fold better reduction in HBV DNA by week 4 than the standard 100 mg

clinical dose of lamivudine. These analytic results indicate that the current clinical dosing of lamivudine achieves only about 10% of the antiviral potential of this anti-HBV agent compared to the optimal dosing levels discovered in our analyses.

**Table 5.** Examples of $E_{max}$ model predicted doses and viral responses at week 4

| Doses (mg/day) | 100 | 300 | 400 | 500 | 600 | 900 | 1200 | 1500 |
|---|---|---|---|---|---|---|---|---|
| HBV DNA reduction ($log_{10}$) | 1.99 | 2.64 | 2.79 | 2.90 | 2.99 | 3.16 | 3.27 | 3.35 |
| Increment over 100 mg | | 0.65 | 0.80 | 0.91 | 1.00 | 1.17 | 1.28 | 1.36 |

*Example 4. Clinical Data*

A physician-initiated pilot clinical observation evaluating the clinical efficacy of an optimal dose of lamivudine in *de novo* combination with a reduced dose of adefovir dipivoxil in HBeAg-positive patients with chronic hepatitis B infection is ongoing. Interim results at 6 month form one patient are summarized in the case report below.

TMS is a 46-year old Chinese male who, during his hospital visit on April 11, 2010, reported that had chronic hepatitis B (CHB) for a number of years and had recently been feeling weak and loosing appetite.

Laboratory tests confirmed that he was positive for serologic markers of HBV infection (HBsAg, HBeAg and HBcAb), and he had evidence of ongoing, clinically-significant HBV replication, reflected by a pre-treatment serum HBV DNA level of $5.37\times10^7$ ($7.73log_{10}$ copies/mL), with evidence of HBV-related liver inflammation indicated by an elevated ALT level of 75 U/mL. The patient was not cirrhotic and had normal renal function.

The patient indicated that he had never been treated with interferon or anti-HBV nucleosides or nucleotides but had been using "liver-protecting" traditional Chinese medicines (TCMs) on an intermittent basis. He was asked to stop taking TCMs and was put on *de novo* combination treatment with 600mg/day lamivudine [6×100mg Heptotin tablets (same as Epivir HBV) manufactured by GlaxoSmithKline] and 5mg/day adefovir dipivoxil [half of 10mg Hepsera tablet manufactured by Gilead Sciences].

Interim results through week 32 indicate that the combination of an optimal dose of lamivudine (600mg/day) and a reduced dose of adefovir dipivoxil (5mg/day) has been highly effective and well-tolerated, with no incidence of nephrotoxicity or other clinically relevant adverse events or laboratory abnormalities reported to date.

Serum HBV DNA declined rapidly following treatment initiation with HB V DNA reductions of 2.12, 3.18 and $4.73log_{10}$ from baseline at weeks 1, 4 and 16 respectively. Residual serum HBV DNA was below $4log_{10}$ copies/mL between week 4 and week 8. With a lower limit of detection of 1000 copies/mL of the in-house PCR assay, serum HBV DNA became PCR negative at week 16 and has remained negative at this interim analysis through week 32, the latest available data **(FIG.** 4).

Resistance analyses were performed for serum samples obtained at baseline and weeks 12 and 24. Sequencing of PCR-amplifieid HBV DNA from the patient's serum samples did not detect any mutations known to be associated with lamivudine or adefovir treatment,

Parallel to the rapid clearance of high levels of serum HBV DNA to PCR-nondetectable levels, in response to treatment with the novel combination of 600mg/day lamivudine and 5mg/day adefovir dipivoxil, serum ALT level fell rapidly to normal levels (upper limit of normal=43U/ml) by week 12, reflecting reduced liver inflammation; and ALT levels have remained normalized in this interim analysis through week 32, the most recent data **(FIG. 5**).

It has been shown that residual viral load at week 4 and most accurately at week 16 was predictive of lamivudine treatment outcome at year 5. Patients with absolute serum HBV DNA levels below 3.6 $log_{10}$ copies/mL (4000 copies/mL or 800IU/mL) at week 16 had a 100% chance to achieve an ideal response, defined as HBV DNA levels of less than 2000 copies/mL (400 IU/mL), HBeAg seroconversion, ALT normalization, and an absence of YMDD mutations after 5 years of treatment (Yuen MF et al. "Hepatitis B virus DNA levels at week 4 of lamivudine treatment predict the 5-year ideal response" Hepatology. 2007, 46(6):1695-1703).

As can be appreciated by those skilled in the art, the patient with HBV DNA below 1000 or $3log_{10}$ copies/mL since week 16 and normalized ALT since week 12 has an excellent prognosis with regard to an enhanced probability of achieving HBeAg seroconversion, the third key clinical benefit desired with antiviral therapy for patients with CHB (in addition to clearance of detectable HBV DNA and reduced liver inflammation reflected by normalized ALT levels). No evidence of resistance has been detected through week 24, the patient's last visit for which HBV DNA sequencing data are available. Therefore, the enhanced viral suppression achieved with an optimal dose of lamivudine in conjunction with a concomitant, reduced dose of adefovir dipivoxil, produced rapid and profound antiviral efficacy with a well-tolerated treatment regimen, while preventing the emergence of resistant viral mutants of either drug.

To our knowledge, this is the first case where a patient with CHB has been treated with the *de novo* combination of an optimal dose of lamivudine (600mg/day) and a reduced dose of adefovir dipivoxil (5mg/day). The clinical benefits

predicted for our optimal combination treatment were evident in the patient's treatment results, as follows: (a) he achieved a rapid and profound viral response, with residual viral load below $4\log_{10}$ copies/mL before week 8 and became PCR negative ($<3\log_{10}$ copies/mL) at week 16; (b) the patient achieved rapid and sustained normalization of his serum ALT level, by week 12; and (c) no evidence of resistance has been detected to date.

In summary, with clinical data extending to 8 months (32 weeks), this physician-initiated pilot clinical observation supports the important treatment benefits predicted for the *de novo* combination of an optimal dose of lamivudine (600mg/day) and a reduced subclinical dose of adefovir dipivoxil (5mg/day).

*References:*

1. Johnson MA, Moore KH, Yuen GJ, Bye A, Pakes GE. Clinical pharmacokinetics of lamivudine. Clin Pharmacokinet. 1999, 36(1):41-66.
2. Yuen MF, Fong DY, Wong DK, Yuen JC, Fung J, Lai CL. Hepatitis B virus DNA levels at week 4 of lamivudine treatment predict the 5-year ideal response. Hepatology. 2007, 46(6):1695-703.
3. Wang CC, Holte S, Huang ML, Sacks SL, Engelberg R, Ferrenberg J, Shuhart M, Corey L. Kinetics of hepatitis B viral load during 48 weeks of treatment with 600 mg vs 100 mg of lamivudine daily. J Viral Hepat, 2004, 11(5):443-7.
4. Torre F, Giannini EG, Basso M, Savarino V, Picciotto A. Initial high dose of lamivudine delays the appearance of viral resistance in chronic hepatitis B patients. Hepatology. 2009, 50(4, suppl):A524-A525.
5. Tsiang M, Rooney JF, Toole JJ, Gibbs CS. Biphasic clearance kinetics of hepatitis B virus from patients during adefovir dipivoxil therapy. Hepatology. 1999, 29(6):1863-1869.
6. Buti M, Esteban R. Entecavir, FTC, L-FMAU, LdT and others. 2003, J Hepatol. 39 S139-S142.
7. Epivir-HBV product label, http://us.gsk.com/products/assets/us_epivir_bbv.pdf.).
8. Hepsera product label, http://www.hepsera.com/pdf/GIL214-07_Hepsera_PDF.8.pdf.
9. Hadziyannis SJ, Tassopoulos NC, Heathcote EJ, Chang TT, Kitis G, Rizzetto M, Marcellin P, Lim SG, Goodman Z, Ma J, Brosgart CL, Borroto-Esoda K, Arterburn S, Chuck SL. Adefovir Dipivoxil 438 Study Group.Long-term therapy with adefovir dipivoxil for HBeAg-negative chronic hepatitis B for up to 5 years. Gastroenterology. 2006, 131(6):1743-1751.
10. Marcellin P, Chang TT, Lim SG, Tong MJ, Sievert W, Shiffman ML, Jeffers L, Goodman Z, Wulfsohn MS, Xiong S, Fry J, Brosgart CL; Adefovir Dipivoxil 437 Study Group. Adefovir dipivoxil for the treatment of hepatitis B e antigen-positive chronic hepatitis B. N Engl J Med. 2003, 348(9):808-816.
11. Marcellin P, Chang TT, Lim SG, Sievert W, Tong M, Arterburn S, Borroto-Esoda K, Frederick D, Rousseau F. Long-tern efficacy and safety of adefovir dipivoxil for the treatment of hepatitis B e antigen-positive chronic hepatitis B. Hepatology. 2008, 48(3):750-758.
12. Lok ASF and McMahon BJ. AASLD practice guidelines. Chronic hepatitis B: update 2009. Hepatology. 2009, 50(3): 1-35.
13. Delaney WE 4th, Yang H, Miller MD, Gibbs CS, Xiong S. Combinations of adefovir with nucleoside analogs produce additive antiviral effects against hepatitis B virus in vitro. Antimicrob Agents Chemother. 2004, 48(10):3702-3710.
14. Colledge D, Civitico G, Locarnini S, Shaw T. In vitro antihepadnaviral activities of combinations of penciclovir, lamivudine, and adefovir. Antimicrob Agents Chemother. 2000, 44(3):551-560.
15. Shaw T. Colledge D, Locarnini S. Synergistic inhibition in vitro of hepadnaviral replication by PMEA and penciclovir or lamivudine. Antiviral Res. 1997, 34(2):A51.
16. Chinese Society of Hepatology, Chinese Medical Association and Chinese Society of Infectious Diseases, Chinese Medical Association. Guideline on prevention and treatment of chronic hepatitis B in China 2005. Chinese Med J. 2007, 120(24):2159-2173.
17. Sung JJ, Lai JY, Zeuzem S, Chow WC, Heathcote EJ, Perrillo RP, Brosgart CL, Woessner MA, Scott SA, Gray DF, Gardner SD. Lamivudine compared with lamivudine and adefovir dipivoxil for the treatment of HBeAg-positive chronic hepatitis B. J Hepatol. 2008, 48(5):728-735.
18. Chan HL, Heathcote EJ, Marcellin P, Lai CL, Cho M, Moon YM, Chao YC, Myers RP, Minuk GY, Jeffers L, Sievert W, Bzowej N, Harb G, Kaiser R, Qiao XJ, Brown NA; 018 Study Group. Treatment of hepatitis B e antigen positive chronic hepatitis with telbivudine or adefovir: a randomized trial. Ann Intern Med. 2007, 147(11):745-754.
19. Korba BE, Gerin JL. Use of a standardized cell culture assay to assess activities of nucleoside analogs against hepatitis B virus replication. Antiviral Res. 1992, 19(1):55-70.

## Claims

1. A pharmaceutical composition for use as a medicament for the treatment of hepatitis B virus infection in a human, comprising:

   a first active pharmaceutical ingredient consisting of more than 400 mg to 1500 mg of lamivudine, (2R, cis)-4-amino-1-(2-hydroxymethyl-1,3-oxathiolan-5-yl)-pyrimidin-2-one, or a therapeutically equivalent amount of a pharmaceutically acceptable salt or ester thereof; and
   a second active pharmaceutical ingredient consisting of 1 mg to 10 mg of adefovir dipivoxil, bis(pivaloyloxymethyl)(9-[(R)-2(phosphonomethoxy)ethyl]adenine, or a therapeutically equivalent amount of a pharmaceutically acceptable salt or ester thereof.

2. The pharmaceutical composition for use according to Claim 1, wherein:

   the first active ingredient consists of more than 400 mg to 900 mg of lamivudine, or a therapeutically equivalent amount of a pharmaceutically acceptable salt or ester thereof, and the second active ingredient consists of 2 mg to 8 mg of adefovir dipivoxil, or a therapeutically equivalent amount of a pharmaceutically acceptable salt or ester thereof;
   the first active ingredient consists of more than 600 mg to 900 mg of lamivudine, or a therapeutically equivalent amount of a pharmaceutically acceptable salt or ester thereof, and the second active ingredient consists of 2 mg to 5 mg of adefovir dipivoxil, or a therapeutically equivalent amount of a pharmaceutically acceptable salt or ester thereof;
   the first active ingredient consists of more than 400 mg to 600 mg of lamivudine, or a therapeutically equivalent amount of a pharmaceutically acceptable salt or ester thereof, and the second active ingredient consists of 5 mg to 8 mg of adefovir dipivoxil, or a therapeutically equivalent amount of a pharmaceutically acceptable salt or ester thereof; or
   the first active ingredient consists of 490 mg to 550 mg of lamivudine, or a therapeutically equivalent amount of a pharmaceutically acceptable salt or ester thereof, and the second active ingredient consists of 2 mg to 8 mg of adefovir dipivoxil, or a therapeutically equivalent amount of a pharmaceutically acceptable salt or ester thereof.

3. The pharmaceutical composition for use according to Claim 1, wherein the first active ingredient consists of 405 mg of lamivudine, and
   wherein the second active ingredient consists of 3 mg of adefovir dipivoxil,
   wherein the second active ingredient consists of 5 mg of adefovir dipivoxil,
   wherein the second active ingredient consists of 8 mg of adefovir dipivoxil, or
   wherein the second active ingredient consists of 10 mg of adefovir dipivoxil

4. The pharmaceutical composition for use according to Claim 1, wherein the first active ingredient consists of 460 mg of lamivudine, and
   wherein the second active ingredient consists of 3 mg of adefovir dipivoxil,
   wherein the second active ingredient consists of 5 mg of adefovir dipivoxil,
   wherein the second active ingredient consists of 8 mg of adefovir dipivoxil, or
   wherein the second active ingredient consists of 10 mg of adefovir dipivoxil.

5. The pharmaceutical composition for use according to Claim 1, wherein the first active ingredient consists of 510 mg of lamivudine, and
   wherein the second active ingredient consists of 3 mg of adefovir dipivoxil,
   wherein the second active ingredient consists of 5 mg of adefovir dipivoxil,
   wherein the second active ingredient consists of 8 mg of adefovir dipivoxil, or
   wherein the second active ingredient consists of 10 mg of adefovir dipivoxil.

6. The pharmaceutical composition for use according to Claim 1, wherein the first active ingredient consists of 550 mg of lamivudine, and
   wherein the second active ingredient consists of 3 mg of adefovir dipivoxil,
   wherein the second active ingredient consists of 4 mg of adefovir dipivoxil,
   wherein the second active ingredient consists of 5 mg of adefovir dipivoxil,
   wherein the second active ingredient consists of 6 mg of adefovir dipivoxil,

wherein the second active ingredient consists of 7 mg of adefovir dipivoxil, or
wherein the second active ingredient consists of 8 mg of adefovir dipivoxil

**7.** The pharmaceutical composition for use according to Claim 1, wherein the first active ingredient consists of 600 mg of lamivudine, and
wherein the second active ingredient consists of 3 mg of adefovir dipivoxil,
wherein the second active ingredient consists of 4 mg of adefovir dipivoxil,
wherein the second active ingredient consists of 5 mg of adefovir dipivoxil,
wherein the second active ingredient consists of 6 mg of adefovir dipivoxil,
wherein the second active ingredient consists of 7 mg of adefovir dipivoxil, or
wherein the second active ingredient consists of 8 mg of adefovir dipivoxil

**8.** The pharmaceutical composition for use according to Claim 1, wherein the first active ingredient consists of 650 mg of lamivudine, and
wherein the second active ingredient consists of 3 mg of adefovir dipivoxil,
wherein the second active ingredient consists of 4 mg of adefovir dipivoxil, or
wherein the second active ingredient consists of 5 mg of adefovir dipivoxil

**9.** The pharmaceutical composition for use according to Claim 1, wherein the first active ingredient consists of 900 mg of lamivudine, and
wherein the second active ingredient consists of 3 mg of adefovir dipivoxil,
wherein the second active ingredient consists of 5 mg of adefovir dipivoxil,
or
wherein the second active ingredient consists of 8 mg of adefovir dipivoxil.

**10.** The pharmaceutical composition for use according to any one of claims 1-9, being in the form of a solid formulation suitable for oral administration.

**11.** The pharmaceutical composition for user according to any of claims 1-10, being in the form of a tablet.

**12.** The pharmaceutical composition for use according to any of claims 1-10, being in the form of a capsule.

**13.** The pharmaceutical composition for use according to any of claims 1-10, being in the form of a solution or suspension suitable for oral administration.

**14.** The pharmaceutical composition for use according to any of claims 1-10, being in the form suitable for parenteral administration.

**15.** A pharmaceutical composition for use according to any one of claims 1-14 comprising one or more pharmaceutically acceptable carriers therefor.

**Patentansprüche**

**1.** Pharmazeutische Zusammensetzung für die Verwendung als Medikament für die Behandlung von Hepatitis-B-Virusinfektion bei einem Menschen, die Folgendes umfasst:

einen ersten pharmazeutischen Wirkstoff, der aus mehr als 400 mg bis 1500 mg Lamivudin, (2R, cis)-4-Amino-1-(2-hydroxymethyl-1,3-oxathiolan-5-yl)pyrimidin-2-on, oder einer therapeutisch äquivalenten Menge eines pharmazeutisch verträglichen Salzes oder Esters davon besteht; und
einen zweiten pharmazeutischen Wirkstoff, der aus 1 mg bis 10 mg Adefovir-Dipivoxil, Bis(pivaloyloxymethyl)-(9-[(R)-2-(phosphonomethoxy)ethyl]adenin, oder einer therapeutisch äquivalenten Menge eines pharmazeutisch verträglichen Salzes oder Esters davon besteht.

**2.** Pharmazeutische Zusammensetzung für die Verwendung nach Anspruch 1, worin:

der erste Wirkstoff aus mehr als 400 mg bis 900 mg Lamivudin oder einer therapeutisch äquivalenten Menge eines pharmazeutisch verträglichen Salzes oder Esters davon besteht und der zweite Wirkstoff aus 2 mg bis

8 mg Adefovir-Dipivoxil oder einer therapeutisch äquivalenten Menge eines pharmazeutisch verträglichen Salzes oder Esters davon besteht;
der erste Wirkstoff aus mehr als 600 mg bis 900 mg Lamivudin oder einer therapeutisch äquivalenten Menge eines pharmazeutisch verträglichen Salzes oder Esters davon besteht und der zweite Wirkstoff aus 2 mg bis 5 mg Adefovir-Dipivoxil oder einer therapeutisch äquivalenten Menge eines pharmazeutisch verträglichen Salzes oder Esters davon besteht;
der erste Wirkstoff aus mehr als 400 mg bis 600 mg Lamivudin oder einer therapeutisch äquivalenten Menge eines pharmazeutisch verträglichen Salzes oder Esters davon besteht und der zweite Wirkstoff aus 5 mg bis 8 mg Adefovir-Dipivoxil oder einer therapeutisch äquivalenten Menge eines pharmazeutisch verträglichen Salzes oder Esters davon besteht; oder
der erste Wirkstoff aus 490 mg bis 550 mg Lamivudin oder einer therapeutisch äquivalenten Menge eines pharmazeutisch verträglichen Salzes oder Esters davon besteht und der zweite Wirkstoff aus 2 mg bis 8 mg Adefovir-Dipivoxil oder einer therapeutisch äquivalenten Menge eines pharmazeutisch verträglichen Salzes oder Esters davon besteht.

**3.** Pharmazeutische Zusammensetzung für die Verwendung nach Anspruch 1, worin der erste Wirkstoff aus 405 mg Lamivudin besteht und
worin der zweite Wirkstoff aus 3 mg Adefovir-Dipivoxil besteht,
worin der zweite Wirkstoff aus 5 mg Adefovir-Dipivoxil besteht,
worin der zweite Wirkstoff aus 8 mg Adefovir-Dipivoxil besteht
oder
worin der zweite Wirkstoff aus 10 mg Adefovir-Dipivoxil besteht.

**4.** Pharmazeutische Zusammensetzung für die Verwendung nach Anspruch 1, worin der erste Wirkstoff aus 460 mg Lamivudin besteht und
worin der zweite Wirkstoff aus 3 mg Adefovir-Dipivoxil besteht,
worin der zweite Wirkstoff aus 5 mg Adefovir-Dipivoxil besteht,
worin der zweite Wirkstoff aus 8 mg Adefovir-Dipivoxil besteht
oder
worin der zweite Wirkstoff aus 10 mg Adefovir-Dipivoxil besteht.

**5.** Pharmazeutische Zusammensetzung für die Verwendung nach Anspruch 1, worin der erste Wirkstoff aus 510 mg Lamivudin besteht und
worin der zweite Wirkstoff aus 3 mg Adefovir-Dipivoxil besteht,
worin der zweite Wirkstoff aus 5 mg Adefovir-Dipivoxil besteht,
worin der zweite Wirkstoff aus 8 mg Adefovir-Dipivoxil besteht
oder
worin der zweite Wirkstoff aus 10 mg Adefovir-Dipivoxil besteht.

**6.** Pharmazeutische Zusammensetzung für die Verwendung nach Anspruch 1, worin der erste Wirkstoff aus 550 mg Lamivudin besteht und
worin der zweite Wirkstoff aus 3 mg Adefovir-Dipivoxil besteht,
worin der zweite Wirkstoff aus 4 mg Adefovir-Dipivoxil besteht,
worin der zweite Wirkstoff aus 5 mg Adefovir-Dipivoxil besteht,
worin der zweite Wirkstoff aus 6 mg Adefovir-Dipivoxil besteht,
worin der zweite Wirkstoff aus 7 mg Adefovir-Dipivoxil besteht oder
worin der zweite Wirkstoff aus 8 mg Adefovir-Dipivoxil besteht.

**7.** Pharmazeutische Zusammensetzung für die Verwendung nach Anspruch 1, worin der erste Wirkstoff aus 600 mg Lamivudin besteht und
worin der zweite Wirkstoff aus 3 mg Adefovir-Dipivoxil besteht,
worin der zweite Wirkstoff aus 4 mg Adefovir-Dipivoxil besteht,
worin der zweite Wirkstoff aus 5 mg Adefovir-Dipivoxil besteht,
worin der zweite Wirkstoff aus 6 mg Adefovir-Dipivoxil besteht,
worin der zweite Wirkstoff aus 7 mg Adefovir-Dipivoxil besteht oder
worin der zweite Wirkstoff aus 8 mg Adefovir-Dipivoxil besteht.

**8.** Pharmazeutische Zusammensetzung für die Verwendung nach Anspruch 1, worin der erste Wirkstoff aus 650 mg

Lamivudin besteht und
worin der zweite Wirkstoff aus 3 mg Adefovir-Dipivoxil besteht,
worin der zweite Wirkstoff aus 4 mg Adefovir-Dipivoxil besteht oder
worin der zweite Wirkstoff aus 5 mg Adefovir-Dipivoxil besteht.

**9.** Pharmazeutische Zusammensetzung für die Verwendung nach Anspruch 1, worin der erste Wirkstoff aus 900 mg Lamivudin besteht und
worin der zweite Wirkstoff aus 3 mg Adefovir-Dipivoxil besteht,
worin der zweite Wirkstoff aus 5 mg Adefovir-Dipivoxil besteht
oder
worin der zweite Wirkstoff aus 8 mg Adefovir-Dipivoxil besteht.

**10.** Pharmazeutische Zusammensetzung für die Verwendung nach einem der Ansprüche 1-9, die in Form einer festen Formulierung vorliegt, die für eine orale Verabreichung geeignet ist.

**11.** Pharmazeutische Zusammensetzung für die Verwendung nach einem der Ansprüche 1-10, die in Form einer Tablette vorliegt.

**12.** Pharmazeutische Zusammensetzung für die Verwendung nach einem der Ansprüche 1-10, die in Form einer Kapsel vorliegt.

**13.** Pharmazeutische Zusammensetzung für die Verwendung nach einem der Ansprüche 1-10, die in Form einer Lösung oder Suspension vorliegt, die für eine orale Verabreichung geeignet ist.

**14.** Pharmazeutische Zusammensetzung für die Verwendung nach einem der Ansprüche 1-10, die in einer Form vorliegt, die für eine parenterale Verabreichung geeignet ist.

**15.** Pharmazeutische Zusammensetzung für die Verwendung nach einem der Ansprüche 1-14, die dafür einen oder mehrere pharmazeutisch verträgliche Träger umfasst.

## Revendications

**1.** Composition pharmaceutique pour une utilisation en tant que médicament dans le traitement de l’infection à virus de l’hépatite B chez un humain, comprenant :

un premier ingrédient pharmaceutique actif consistant en de plus de 400 mg à 1 500 mg de lamivudine (2*R*, *cis*)-4-amino-1-(2-hydroxyméthyl-1,3-oxathiolan-5-yl)-pyrimidin-2-one ou en une quantité thérapeutiquement équivalente d’un sel ou d’un ester pharmaceutiquement acceptable de celle-ci ; et
un second ingrédient pharmaceutique actif consistant en de 1 mg à 10 mg d’adéfovir dipivoxil bis(pivaloyloxyméthyl)(9-[(*R*)-2(phosphonométhoxy)éthyl]adénine ou en une quantité thérapeutiquement équivalente d’un sel ou d’un ester pharmaceutiquement acceptable de celui-ci.

**2.** Composition pharmaceutique pour une utilisation selon la revendication 1, dans laquelle :

le premier ingrédient actif consiste en de plus de 400 mg à 900 mg de lamivudine ou en une quantité thérapeutiquement équivalente d’un sel ou d’un ester pharmaceutiquement acceptable de celle-ci et le second ingrédient actif consiste en de 2 mg à 8 mg d’adéfovir dipivoxil ou en une quantité thérapeutiquement équivalente d’un sel ou d’un ester pharmaceutiquement acceptable de celui-ci ;
le premier ingrédient actif consiste en de plus de 600 mg à 900 mg de lamivudine ou en une quantité thérapeutiquement équivalente d’un sel ou d’un ester pharmaceutiquement acceptable de celle-ci et le second ingrédient actif consiste en de 2 mg à 5 mg d’adéfovir dipivoxil ou en une quantité thérapeutiquement équivalente d’un sel ou d’un ester pharmaceutiquement acceptable de celui-ci ;
le premier ingrédient actif consiste en de plus de 400 mg à 600 mg de lamivudine ou en une quantité thérapeutiquement équivalente d’un sel ou d’un ester pharmaceutiquement acceptable de celle-ci et le second ingrédient actif consiste en de 5 mg à 8 mg d’adéfovir dipivoxil ou en une quantité thérapeutiquement équivalente d’un sel ou d’un ester pharmaceutiquement acceptable de celui-ci ; ou
le premier ingrédient actif consiste en de 490 mg à 550 mg de lamivudine ou en une quantité thérapeutiquement

équivalente d'un sel ou d'un ester pharmaceutiquement acceptable de celle-ci et le second ingrédient actif consiste en de 2 mg à 8 mg d'adéfovir dipivoxil ou en une quantité thérapeutiquement équivalente d'un sel ou d'un ester pharmaceutiquement acceptable de celui-ci.

**3.** Composition pharmaceutique pour une utilisation selon la revendication 1, dans laquelle le premier ingrédient actif consiste en 405 mg de lamivudine et
dans laquelle le second ingrédient actif consiste en 3 mg d'adéfovir dipivoxil,
dans laquelle le second ingrédient actif consiste en 5 mg d'adéfovir dipivoxil,
dans laquelle le second ingrédient actif consiste en 8 mg d'adéfovir dipivoxil ou
dans laquelle le second ingrédient actif consiste en 10 mg d'adéfovir dipivoxil.

**4.** Composition pharmaceutique pour une utilisation selon la revendication 1, dans laquelle le premier ingrédient actif consiste en 460 mg de lamivudine et
dans laquelle le second ingrédient actif consiste en 3 mg d'adéfovir dipivoxil,
dans laquelle le second ingrédient actif consiste en 5 mg d'adéfovir dipivoxil,
dans laquelle le second ingrédient actif consiste en 8 mg d'adéfovir dipivoxil ou
dans laquelle le second ingrédient actif consiste en 10 mg d'adéfovir dipivoxil.

**5.** Composition pharmaceutique pour une utilisation selon la revendication 1, dans laquelle le premier ingrédient actif consiste en 510 mg de lamivudine et
dans laquelle le second ingrédient actif consiste en 3 mg d'adéfovir dipivoxil,
dans laquelle le second ingrédient actif consiste en 5 mg d'adéfovir dipivoxil,
dans laquelle le second ingrédient actif consiste en 8 mg d'adéfovir dipivoxil ou
dans laquelle le second ingrédient actif consiste en 10 mg d'adéfovir dipivoxil.

**6.** Composition pharmaceutique pour une utilisation selon la revendication 1, dans laquelle le premier ingrédient actif consiste en 550 mg de lamivudine et
dans laquelle le second ingrédient actif consiste en 3 mg d'adéfovir dipivoxil,
dans laquelle le second ingrédient actif consiste en 4 mg d'adéfovir dipivoxil,
dans laquelle le second ingrédient actif consiste en 5 mg d'adéfovir dipivoxil,
dans laquelle le second ingrédient actif consiste en 6 mg d'adéfovir dipivoxil,
dans laquelle le second ingrédient actif consiste en 7 mg d'adéfovir dipivoxil ou
dans laquelle le second ingrédient actif consiste en 8 mg d'adéfovir dipivoxil.

**7.** Composition pharmaceutique pour une utilisation selon la revendication 1, dans laquelle le premier ingrédient actif consiste en 600 mg de lamivudine et
dans laquelle le second ingrédient actif consiste en 3 mg d'adéfovir dipivoxil,
dans laquelle le second ingrédient actif consiste en 4 mg d'adéfovir dipivoxil,
dans laquelle le second ingrédient actif consiste en 5 mg d'adéfovir dipivoxil,
dans laquelle le second ingrédient actif consiste en 6 mg d'adéfovir dipivoxil,
dans laquelle le second ingrédient actif consiste en 7 mg d'adéfovir dipivoxil ou
dans laquelle le second ingrédient actif consiste en 8 mg d'adéfovir dipivoxil.

**8.** Composition pharmaceutique pour une utilisation selon la revendication 1, dans laquelle le premier ingrédient actif consiste en 650 mg de lamivudine et
dans laquelle le second ingrédient actif consiste en 3 mg d'adéfovir dipivoxil,
dans laquelle le second ingrédient actif consiste en 4 mg d'adéfovir dipivoxil ou
dans laquelle le second ingrédient actif consiste en 5 mg d'adéfovir dipivoxil.

**9.** Composition pharmaceutique pour une utilisation selon la revendication 1, dans laquelle le premier ingrédient actif consiste en 900 mg de lamivudine et
dans laquelle le second ingrédient actif consiste en 3 mg d'adéfovir dipivoxil,
dans laquelle le second ingrédient actif consiste en 5 mg d'adéfovir dipivoxil ou
dans laquelle le second ingrédient actif consiste en 8 mg d'adéfovir dipivoxil.

**10.** Composition pharmaceutique pour une utilisation selon l'une quelconque des revendications 1-9 qui est sous la forme d'une formulation solide se prêtant à une administration orale.

**11.** Composition pharmaceutique pour une utilisation selon l'une quelconque des revendications 1-10 qui est sous la forme d'un comprimé.

**12.** Composition pharmaceutique pour une utilisation selon l'une quelconque des revendications 1-10 qui est sous la forme d'une gélule.

**13.** Composition pharmaceutique pour une utilisation selon l'une quelconque des revendications 1-10 qui est sous la forme d'une solution ou d'une suspension se prêtant à une administration orale.

**14.** Composition pharmaceutique pour une utilisation selon l'une quelconque des revendications 1-10 qui est sous une forme se prêtant à une administration parentérale.

**15.** Composition pharmaceutique pour une utilisation selon l'une quelconque des revendications 1-14 qui comprend un ou plusieurs véhicules pharmaceutiquement acceptables pour celle-ci.

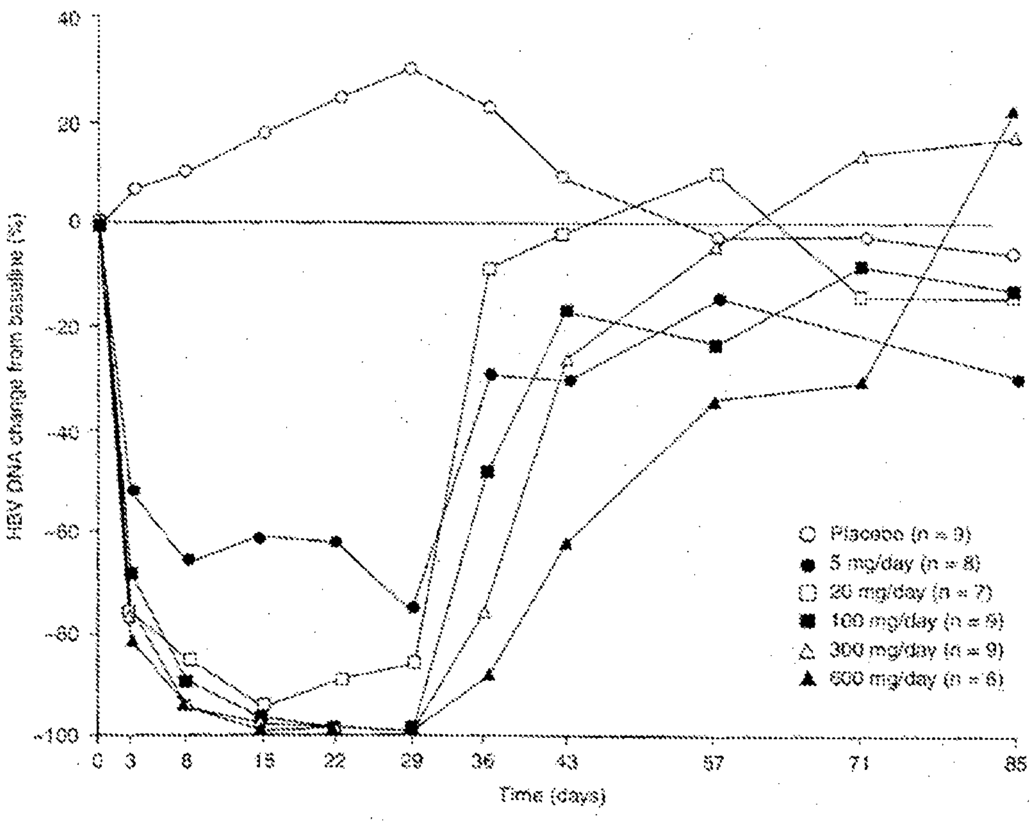


**FIG. 1**. From Clinical Pharmacokinetics, 26(14):41-66

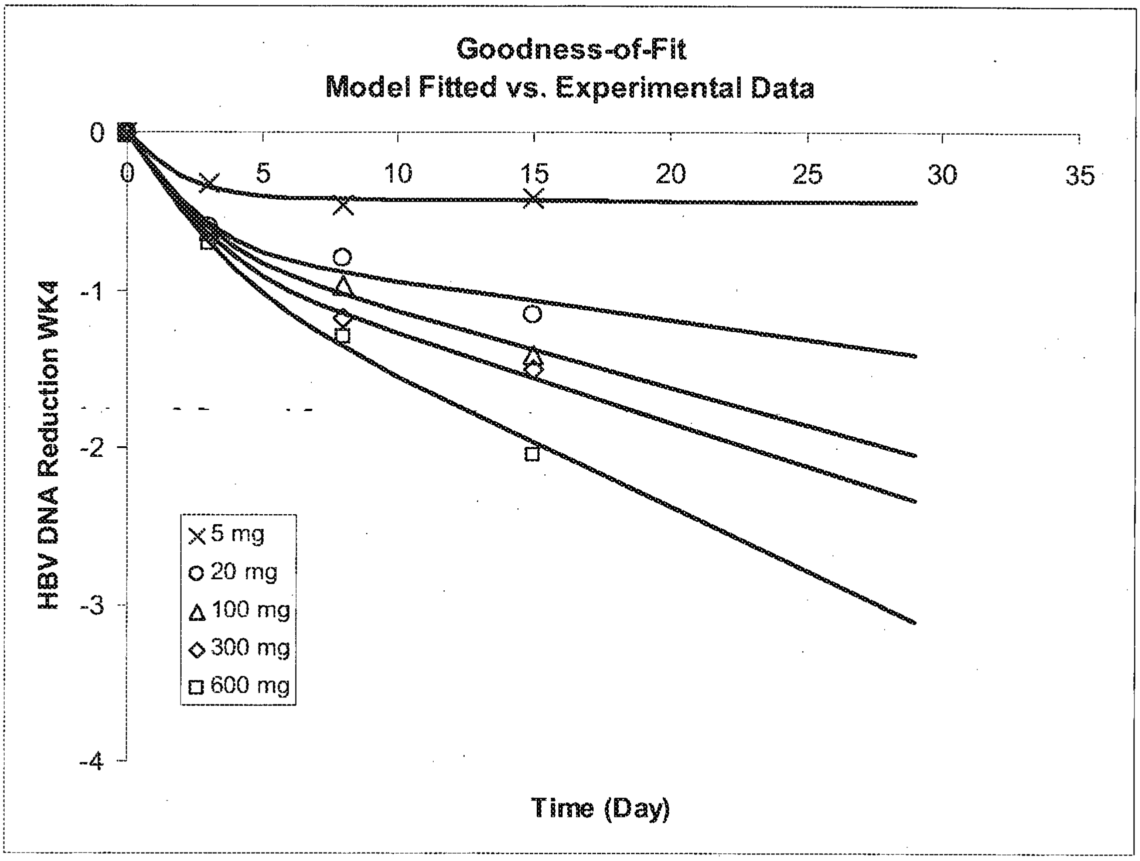


FIG. 2. Simulated viral dynamic profiles of 4 weeks of lamivudine treatment at 5-600 mg/day

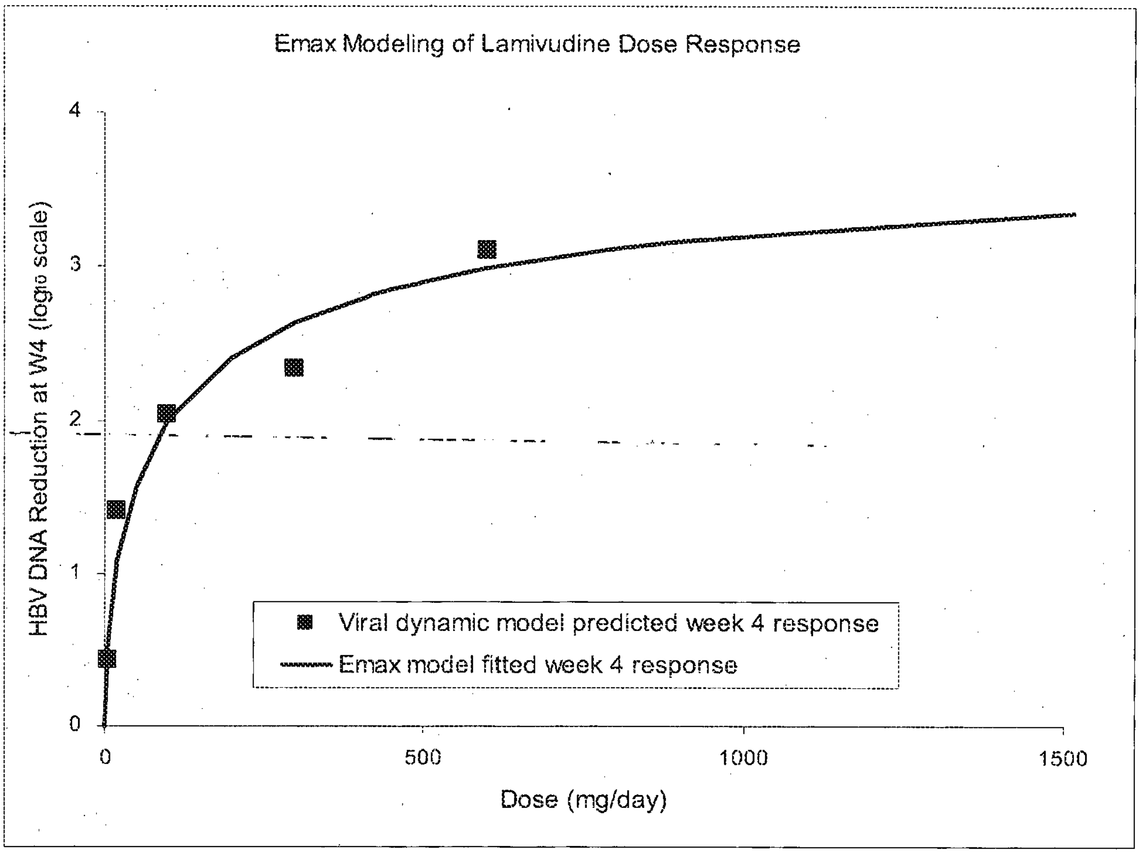


**FIG. 3.** Lamivudine dose optimization by Emax modeling

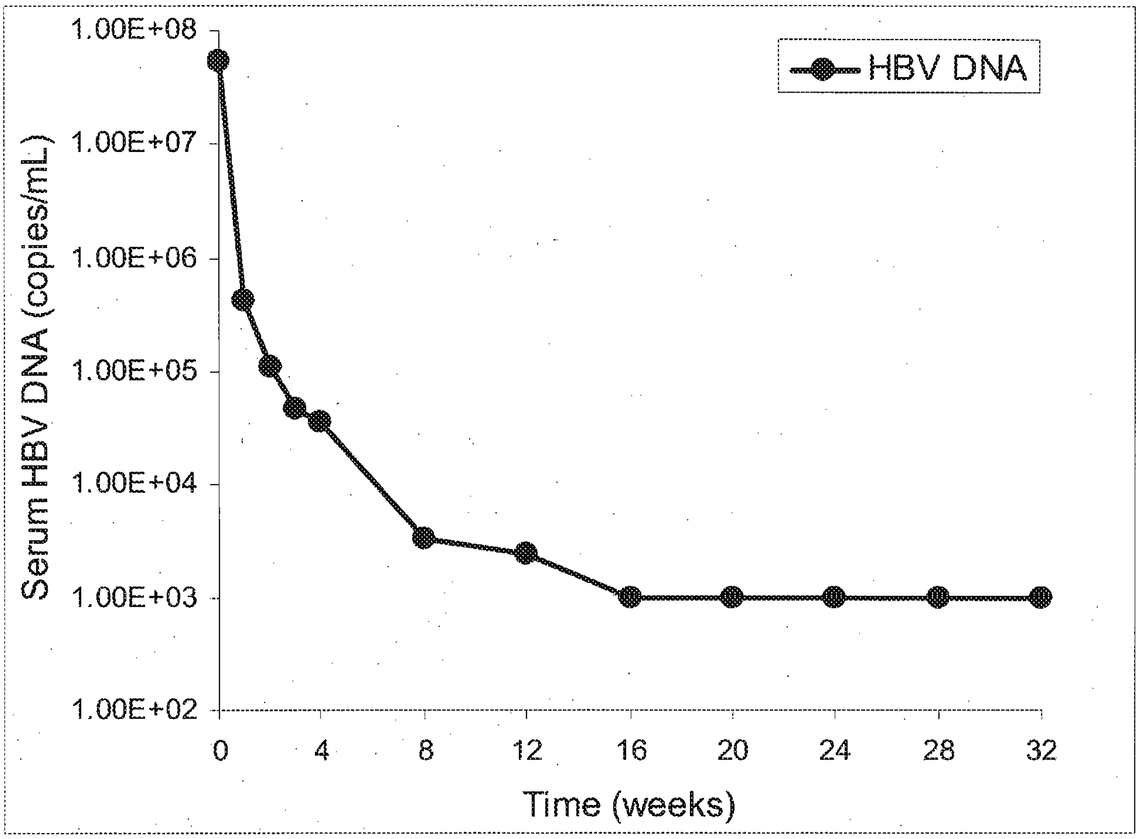


**FIG.4.** Time course of serum HBV DNA in patient TMS following treatment initiation with *de novo* combination of lamivudine 600mg/day and adefovir dipivoxil 5mg/day

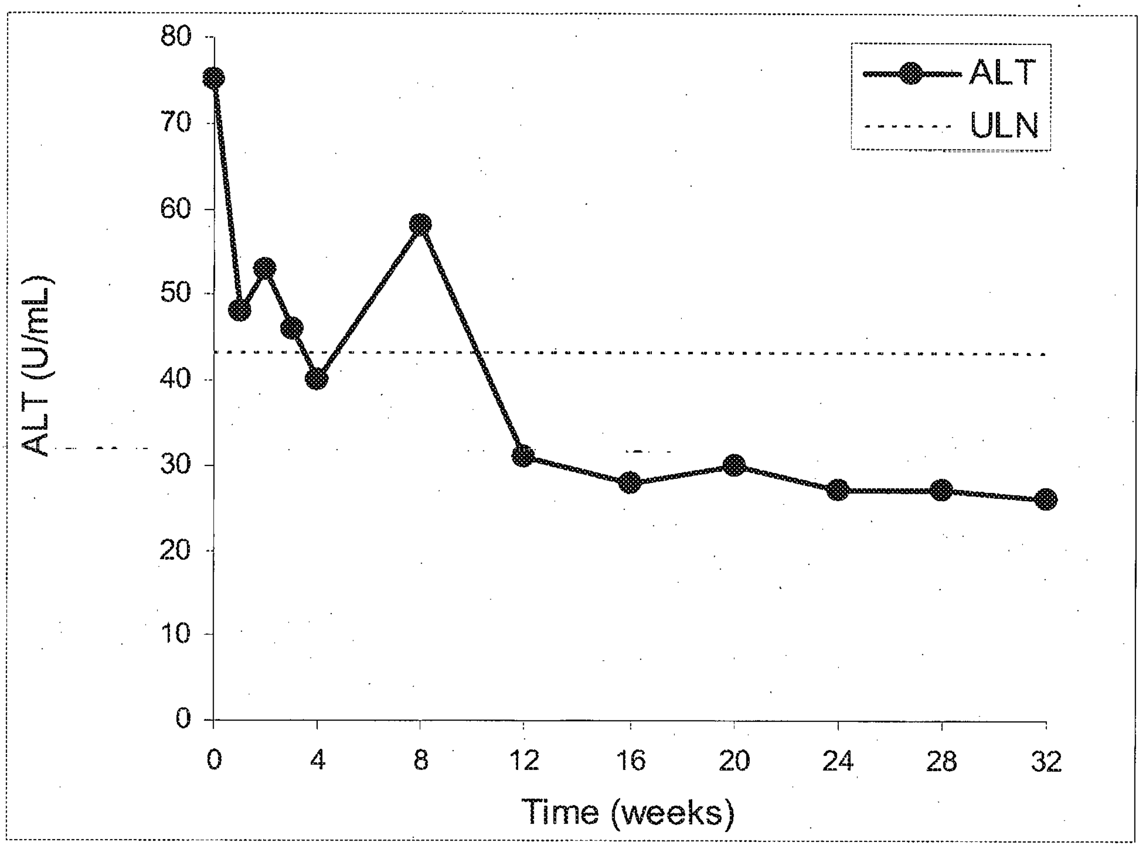


**FIG.5.** Time course of serum ALT in patient TMS following treatment initiation with *de novo* combination of lamivudine 600mg/day and adefovir dipivoxil 5mg/daym (ULN: upper limit of normal)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0016755 A
- US 3119742 A
- US 3492397 A
- US 3538214 A
- US 4060598 A
- US 4173626 A


### Non-patent literature cited in the description


- **CHANG, M.** Hepatitis B virus infection. *Seminars in fetal & neonatal medicine,* 2007, vol. 12 (3), 160-167
- **LIAW.** *J. Clinical Virology,* 2005, vol. 34 (1), S143-S146
- **MARCELLIN et al.** Long-term efficacy and safety of adefovir dipivoxil for the treatment of hepatitis B e antigen-positive chronic hepatitis B. *Hepatology,* 2008, vol. 48 (3), 750-758
- **HADZIYANNIS et al.** Adefovir Dipivoxil 438 Study Group. Long-term therapy with adefovir dipivoxil for HBeAg-negative chronic hepatitis B for up to 5 years. *Gastroenterology,* 2006, vol. 131 (6), 1743-1751
- **LOK et al.** AASLD practice guidelines. Chronic hepatitis B: update 2009. *Hepatology,* 2009, vol. 50 (3), 1-35
- **LOK et al.** AASLD practice guidelines. Chronic hepatitis B: update 2009. *Hepatology,* 2009, vol. 50 (3), 1-35
- **JOHNSON et al.** Clinical pharmacokinetics of lamivudine. *Clin Pharmacokinet,* 1999, vol. 36 (1), 41-66
- **YUEN et al.** Hepatitis B virus DNA levels at week 4 of lamivudine treatment predict the 5-year ideal response. *Hepatology,* 2007, vol. 46 (6), 1695-703
- *European Association for the Study of Liver (EASL) practice guidelines on chronic hepatitis B,* 2008
- **WANG et al.** Kinetics of hepatitis B viral load during 48 weeks of treatment with 600 mg vs. 100 mg of lamivudine daily. *J. Viral Hepat.,* 2004, vol. 11 (5), 443-7
- **TORRE et al.** Initial high dose of lamivudine delays the appearance of viral resistance in chronic hepatitis B patients. *Hepatology,* 2009, vol. 50 (4), A524-A525
- **SHAW et al.** Synergistic inhibition in vitro of hepadnaviral replication by PMEA and penciclovir or lamivudine. *Antiviral Res.,* 1997, vol. 34 (2), A51
- **COLLEDGE et al.** In vitro antihepadnaviral activities of combinations of penciclovir, lamivudine, and adefovir. *Antimicrob Agents Chemother,* 2000, vol. 44 (3), 551-560
- **DELANEY et al.** Combinations of adefovir with nucleoside analogs produce additive antiviral effects against hepatitis B virus in vitro. *Antimicrob Agents Chemother,* 2004, vol. 48 (10), 3702-3710
- **SUNG et al.** Lamivudine compared with lamivudine and adefovir dipivoxil for the treatment of HBeAg-positive chronic hepatitis B. *J. Hepatol.,* 2008, vol. 48 (5), 728-735
- Remington's Pharmaceutical Sciences. Mack Publishing Co, 1985
- **JOHNSON et al.** clinical pharmacokinetics of lamivudine. *Clin. Pharmacokinet,* 1999, vol. 36 (1), 41-66
- **TSIANG et al.** Biphasic clearance kinetics of hepatitis B virus from patients during adefovir dipivoxil therapy. *Hepatology,* 1999, vol. 29 (6), 1863-1869
- **WANG et al.** Kinetics of hepatitis B viral load during 48 weeks of treatment with 600 mg vs 100 mg of lamivudine daily. *J. Viral Hepat.,* 2004, vol. 11 (5), 443-7
- **BUTI et al.** *J. Hepatol.,* 2003, vol. 39, S139-S142
- **YUEN MF et al.** Hepatitis B virus DNA levels at week 4 of lamivudine treatment predict the 5-year ideal response. *Hepatology,* 2007, vol. 46 (6), 1695-1703
- **JOHNSON MA ; MOORE KH ; YUEN GJ ; BYE A ; PAKES GE.** Clinical pharmacokinetics of lamivudine. *Clin Pharmacokinet.,* 1999, vol. 36 (1), 41-66
- **YUEN MF ; FONG DY ; WONG DK ; YUEN JC ; FUNG J ; LAI CL.** Hepatitis B virus DNA levels at week 4 of lamivudine treatment predict the 5-year ideal response. *Hepatology,* 2007, vol. 46 (6), 1695-703

- **WANG CC ; HOLTE S ; HUANG ML ; SACKS SL ; ENGELBERG R ; FERRENBERG J ; SHUHART M ; COREY L.** Kinetics of hepatitis B viral load during 48 weeks of treatment with 600 mg vs 100 mg of lamivudine daily. *J Viral Hepat,* 2004, vol. 11 (5), 443-7
- **TORRE F ; GIANNINI EG ; BASSO M ; SAVARINO V ; PICCIOTTO A.** Initial high dose of lamivudine delays the appearance of viral resistance in chronic hepatitis B patients. *Hepatology,* 2009, vol. 50 (4), A524-A525
- **TSIANG M ; ROONEY JF ; TOOLE JJ ; GIBBS CS.** Biphasic clearance kinetics of hepatitis B virus from patients during adefovir dipivoxil therapy. *Hepatology,* 1999, vol. 29 (6), 1863-1869
- **BUTI M ; ESTEBAN R. ENTECAVIR.** *J Hepatol.,* 2003, vol. 39, S139-S142
- **HADZIYANNIS SJ ; TASSOPOULOS NC ; HEATHCOTE EJ ; CHANG TT ; KITIS G ; RIZZETTO M ; MARCELLIN P ; LIM SG ; GOODMAN Z ; MA J.** Adefovir Dipivoxil 438 Study Group.Long-term therapy with adefovir dipivoxil for HBeAg-negative chronic hepatitis B for up to 5 years. *Gastroenterology,* 2006, vol. 131 (6), 1743-1751
- **MARCELLIN P ; CHANG TT ; LIM SG ; TONG MJ ; SIEVERT W ; SHIFFMAN ML ; JEFFERS L ; GOODMAN Z ; WULFSOHN MS ; XIONG S.** Adefovir Dipivoxil 437 Study Group. Adefovir dipivoxil for the treatment of hepatitis B e antigen-positive chronic hepatitis B. *N Engl J Med.,* 2003, vol. 348 (9), 808-816
- **MARCELLIN P ; CHANG TT ; LIM SG ; SIEVERT W ; TONG M ; ARTERBURN S ; BORROTO-ESODA K ; FREDERICK D ; ROUSSEAU F.** Long-tern efficacy and safety of adefovir dipivoxil for the treatment of hepatitis B e antigen-positive chronic hepatitis B. *Hepatology,* 2008, vol. 48 (3), 750-758
- **LOK ASF ; MCMAHON BJ.** AASLD practice guidelines. Chronic hepatitis B: update 2009. *Hepatology,* 2009, vol. 50 (3), 1-35
- **DELANEY WE ; YANG H ; MILLER MD ; GIBBS CS ; XIONG S.** Combinations of adefovir with nucleoside analogs produce additive antiviral effects against hepatitis B virus in vitro. *Antimicrob Agents Chemother.,* 2004, vol. 48 (10), 3702-3710
- **COLLEDGE D ; CIVITICO G ; LOCARNINI S ; SHAW T.** In vitro antihepadnaviral activities of combinations of penciclovir, lamivudine, and adefovir. *Antimicrob Agents Chemother.,* 2000, vol. 44 (3), 551-560
- **SHAW T ; COLLEDGE D ; LOCARNINI S.** Synergistic inhibition in vitro of hepadnaviral replication by PMEA and penciclovir or lamivudine. *Antiviral Res.,* 1997, vol. 34 (2), A51
- Guideline on prevention and treatment of chronic hepatitis B in China 2005. Chinese Med J. Chinese Medical Association, 2007, vol. 120, 2159-2173
- **SUNG JJ ; LAI JY ; ZEUZEM S ; CHOW WC ; HEATHCOTE EJ ; PERRILLO RP ; BROSGART CL ; WOESSNER MA ; SCOTT SA ; GRAY DF.** Lamivudine compared with lamivudine and adefovir dipivoxil for the treatment of HBeAg-positive chronic hepatitis B. *J Hepatol.,* 2008, vol. 48 (5), 728-735
- **CHAN HL ; HEATHCOTE EJ ; MARCELLIN P ; LAI CL ; CHO M ; MOON YM ; CHAO YC ; MYERS RP ; MINUK GY ; JEFFERS L.** 018 Study Group. Treatment of hepatitis B e antigen positive chronic hepatitis with telbivudine or adefovir: a randomized trial. *Ann Intern Med.,* 2007, vol. 147 (11), 745-754
- **KORBA BE ; GERIN JL.** Use of a standardized cell culture assay to assess activities of nucleoside analogs against hepatitis B virus replication. *Antiviral Res.,* 1992, vol. 19 (1), 55-70